# EUROPEAN PATENT SPECIFICATION

(11) **EP 3 057 617 B1**
(45) Date of publication and mention of the grant of the patent: **01.04.2020**
(21) Application number: 14854108.9
(22) Date of filing: 20.10.2014
(51) Int. Cl.: A61K 9/06, A61K 9/14, A61K 47/42, A61L 27/52, A61K 33/24, A61K 33/243, A61K 33/26, A61K 33/34, A61K 33/38, C07K 7/06

(54) **NANOPARTICLE-CONTAINING HYDROGELS**
NANOPARTIKELHALTIGE HYDROGELE
HYDROGELS CONTENANT DES NANOPARTICULES

(30) Priority: 18.10.2013 SG 201307796
(43) Date of publication of application: 24.08.2016
(73) Proprietor: Agency for Science, Technology and Research, Singapore 138632 (SG)
(72) Inventor: HAUSER, Charlotte A. E., Singapore 138669 (SG); CHIN, Jia Min, Singapore 117602 (SG); REITHOFER, Michael R., Singapore 138669 (SG)
(74) Representative: Smith, Julian Philip Howard
(86) International application number: PCT/SG2014/000493
(87) International publication number: WO 2015/057170

(56) References cited:
- WO-A1-2013/066274
- WO-A1-2013/126017
- US-A1- 2013 267 455
- BIMALENDU ADHIKARI ET AL: "Short-Peptide-Based Hydrogel: A Template for the In Situ Synthesis of Fluorescent Silver Nanoclusters by Using Sunlight", CHEMISTRY - A EUROPEAN JOURNAL, vol. 16, no. 46, 13 October 2010 (2010-10-13), pages 13698-13705, XP55339362, ISSN: 0947-6539, DOI: 10.1002/chem.201001240
- RAJENDRA NARAYAN MITRA ET AL: "In situ Preparation of Gold Nanoparticles of Varying Shape in Molecular Hydrogel of Peptide Amphiphiles", JOURNAL OF PHYSICAL CHEMISTRY C, vol. 112, no. 22, 1 June 2008 (2008-06-01) , pages 8159-8166, XP055388977, US ISSN: 1932-7447, DOI: 10.1021/jp712106d
- ARCHANA MISHRA ET AL: "Ultrasmall natural peptides self-assemble to strong temperature-resistant helical fibers in scaffolds suitable for tissue engineering", NANO TODAY, vol. 6, no. 3, 2 June 2011 (2011-06-02), pages 232-239, XP055066828, ISSN: 1748-0132, DOI: 10.1016/j.nantod.2011.05.001
- ADHIKARI, B . ET AL.: 'Short-Peptide-Based Hydrogel: A Template for the In Situ Synthesis of Fluorescent Silver Nanoclusters by Using Sunlight' CHEM. EUR. J vol. 16, 2010, pages 13698 - 13705, XP055339362
- CHENG, G. ET AL.: 'Hydrogelation and Self-Assembly of Fmoc-Tripeptides: Unexpected Influence of Sequence on Self-Assembled Fibril Structure, and Hydrogel Modulus and Anisotropy' LANGMUIR vol. 26, no. 7, 2010, pages 4990 - 4998, XP055339367
- HAUSER, C.A.E. ET AL.: 'Natural tri- to hexapeptides self-assemble in water to amyloid beta-type fiber aggregates by unexpected a-helical intermediate structures' PNAS vol. 108, no. 4, 2011, pages 1361 - 1366, XP055082546
- LAKSHMANAN, A. ET AL.: 'Ultrasmall Peptides Self-Assemble into Diverse Nanostructures: Morphological Evaluation and Potential Implications' INT. J. MOL. SCI. vol. 12, 2011, pages 5736 - 5746, XP055082543
- SINGH, R. ET AL.: 'Radiation synthesis of PVP/alginate hydrogel containing nanosilver as wound dressing' J MATER SCI: MATER MED vol. 23, 2012, pages 2649 - 2658, XP035135305
- MOHAN, Y. M. ET AL.: 'Controlling of silver nanoparticles structure by hydrogel networks' JOURNAL OF COLLOID AND INTERFACE SCIENCE vol. 342, 2010, pages 73 - 82, XP026806701
- REITHOFER, M. R. ET AL.: 'In situ synthesis of size-controlled, stable silver nanoparticles within ultrashort peptide hydrogels and their anti-bacterial properties' BIOMATERIALS vol. 35, 2014, pages 7535 - 7542, XP028874862

### SEQUENCE LISTING

<110> AGENCY FOR SCIENCE, TECHNOLOGY AND RESEARCH
<120> NANOPARTICLE-CONTAINING HYDROGELS
<130> 9869SG2751
<160> 22
<170> PatentIn version 3.5
<210> 1
   <211> 5
   <212> PRT
   <213> Artificial Sequence
<220>
   <223> SYNTHETIC PEPTIDE
<400> 1
   Leu Ile Val Ala Gly
   1               5
<210> 2
   <211> 5
   <212> PRT
   <213> Artificial Sequence
<220>
   <223> SYNTHETIC PEPTIDE
<400> 2
   Ile Leu Val Ala Gly
   1               5
<210> 3
   <211> 5
   <212> PRT
   <213> Artificial Sequence
<220>
   <223> SYNTHETIC PEPTIDE
<400> 3
   Leu Ile Val Ala Ala
   1               5
<210> 4
   <211> 5
   <212> PRT
   <213> Artificial Sequence
<220>
   <223> SYNTHETIC PEPTIDE
<400> 4
   Leu Ala Val Ala Gly
   1               5
<210> 5
   <211> 4
   <212> PRT
   <213> Artificial Sequence
<220>
   <223> SYNTHETIC PEPTIDE
<400> 5
   Ile Val Ala Gly
   1
<210> 6
   <211> 4
   <212> PRT
   <213> Artificial Sequence
<220>
   <223> SYNTHETIC PEPTIDE
<400> 6
   Leu Ile Val Ala
   1
<210> 7
   <211> 4
   <212> PRT
   <213> Artificial Sequence
<220>
   <223> SYNTHETIC PEPTIDE
<400> 7
   Leu Ile Val Gly
   1
<210> 8
   <211> 6
   <212> PRT
   <213> Artificial Sequence
<220>
   <223> SYNTHETIC PEPTIDE
<400> 8
   Leu Ile Val Ala Gly Asp
   1 5
<210> 9
   <211> 6
   <212> PRT
   <213> Artificial Sequence
<220>
   <223> SYNTHETIC PEPTIDE
<400> 9
   Ile Leu Val Ala Gly Asp
   1 5
<210> 10
   <211> 6
   <212> PRT
   <213> Artificial Sequence
<220>
   <223> SYNTHETIC PEPTIDE
<400> 10
   Leu Ile Val Ala Ala Asp
   1 5
<210> 11
   <211> 6
   <212> PRT
   <213> Artificial Sequence
<220>
   <223> SYNTHETIC PEPTIDE
<400> 11
   Leu Ala Val Ala Gly Asp
   1 5
<210> 12
   <211> 6
   <212> PRT
   <213> Artificial Sequence
<220>
   <223> SYNTHETIC PEPTIDE
<400> 12
   Ala Ile Val Ala Gly Asp
   1 5
<210> 13
   <211> 6
   <212> PRT
   <213> Artificial Sequence
<220>
   <223> SYNTHETIC PEPTIDE
<400> 13
   Leu Ile Val Ala Gly Glu
   1 5
<210> 14
   <211> 6
   <212> PRT
   <213> Artificial Sequence
<220>
   <223> SYNTHETIC PEPTIDE
<400> 14
   Leu Ile Val Ala Gly Lys
   1 5
<210> 15
   <211> 6
   <212> PRT
   <213> Artificial Sequence
<220>
   <223> SYNTHETIC PEPTIDE
<400> 15
   Ile Leu Val Ala Gly Lys
   1 5
<210> 16
   <211> 6
   <212> PRT
   <213> Artificial Sequence
<220>
   <223> SYNTHETIC PEPTIDE
<400> 16
   Leu Ile Val Ala Gly Thr
   1 5
<210> 17
   <211> 6
   <212> PRT
   <213> Artificial Sequence
<220>
   <223> SYNTHETIC PEPTIDE
<400> 17
   Ala Ile Val Ala Gly Thr
   1 5
<210> 18
   <211> 5
   <212> PRT
   <213> Artificial Sequence
<220>
   <223> SYNTHETIC PEPTIDE
<400> 18
   Leu Ile Val Ala Asp
   1 5
<210> 19
   <211> 5
   <212> PRT
   <213> Artificial sequence
<220>
   <223> SYNTHETIC PEPTIDE
<400> 19
   Leu Ile Val Gly Asp
   1 5
<210> 20
   <211> 4
   <212> PRT
   <213> Artificial Sequence
<220>
   <223> SYNTHETIC PEPTIDE
<400>20
   Ile Val Ala Asp
   1
<210> 21
   <211> 4
   <212> PRT
   <213> Artificial Sequence
<220>
   <223> SYNTHETIC PEPTIDE
<400> 21
   Ile Ile Ile Asp
   1
<210> 22
   <211> 4
   <212> PRT
   <213> Artificial Sequence
<220>
   <223> SYNTHETIC PEPTIDE
<400> 22
   Ile Ile Ile Lys
   1

## Description

### Technical Field

The present invention generally relates to nanoparticle-containing hydrogels, compositions thereof, methods for their preparation, and uses thereof. The nanoparticle-containing hydrogels may be useful as an antimicrobial and/or antibacterial agent.

### Background

In recent years the interest in biomaterials possessing inherent antimicrobial activity has increased and a number of different applications including topical applications for the prevention of infections associated with large wounds and prevention of biofilm formation in medical devices have been investigated. Due to the increase in antibiotic-resistant bacteria, the interest in the development of biomaterials containing antibacterial-agents such as silver has grown.

Silver compounds such as silver nitrate solution have been used traditionally in the treatment of human diseases such as gonococcal eye infection, burns and wounds (Kim *et al.,* 2007; Klasen *et al.,* 2000-a; Klasen *et al.,* 2000-b). Silver compounds possess certain advantages over classical antibiotics, such as a broad spectrum of anti-microbial activity against as many as twelve gram-positive and gram-negative bacteria (Leaper, 2006). Further, the existence of multiple cellular targets within the microbes lowers the propensity of inducing microbial resistance to silver-based treatments. Silver-based materials which are highly active against antibiotic-resistant bacterial strains have been developed. In particular, biomaterials impregnated with silver have been tested for clinical use and several products containing either Ag(I) or silver nanoparticles (Ag NPs) are currently available commercially (Leaper, 2006; Alt *et al.,* 2004; Wright *et al.,* 2002). In this context, it should be noted that a recent study compared the anti-microbial effectiveness of various silver compounds, such as silver sulfadiazine and silver nanoparticles (Brandt *et al.,* 2012). Interestingly, it was concluded that the bactericidal properties of silver nanoparticles are superior to other silver compounds and that their potencies increase with a decrease in their sizes (Martinez-Gutierrez *et al.,* 2012). Martinez-Gutierrez *et al.* (2010) specifically report that silver nanoparticles of size 20 to 25 nm were most effective in suppressing the growth of clinically relevant bacteria with moderate to high antibiotic resistance.

Several attempts have been made to develop hydrogels which contain silver but thus far none of these biomaterials are commercially available. Recently Messersmith *et al.* reported water-soluble polyethylene glycol (PEG) polymers containing reactive catechol moieties that can reduce Ag(I) to Ag(0) NP (Fullenkamp *et al.,* 2012). Varaprasad *et al.* (2010) reported the *in situ* reduction of silver in hydrogels using sodium borohydride resulting in the formation of colloidal silver in the hydrogels. In both reported methods, the reduction of silver takes place during polymer gelation. A different method was employed by Murali *et al.* (2010) who reported the controlled formation of silver nanoparticles inside a pre-formed hydrogel network. Furthermore, Banerjee *et al.* reported UV light-induced reduction of silver nitrate using Fmoc protected dipeptides or single amino acids (Adhikari *et al.,* 2010; Roy & Banerjee, 2011).

Two silver-containing wound management products are commercially available - Silvazine™ and Acticoat™. Silvazine™ contains 1% silver sulphadiazine cream with 0.2% chlorhexidine and is used as a burn wound therapeutic product. Acticoat™ has a range of products under its series such as Acticoat™ 7 wherein the use of silver nanocrystalline technology confers its product range their anti-microbial properties. However, these current commercially available products are deficient in some conditions for optimal wound management, increasing the need to search for a more effective wound management candidate.

For an effective wound control, several criteria are necessary. Firstly, wound therapy agents need to provide a moist environment for cells to renew as this has shown to accelerate epithelisation. However, Silvazine™, Acticoat™ 7 and Acticoat™ Flex require external moistening and are unable to provide a moist environment for a sustained period of time.

Secondly, wound therapy agents should also possess anti-microbial properties, preferably sustained over several days, to prevent infection. However, the Acticoat™ products are only capable of offering up to 7 days of anti-microbial protection.

Thirdly, wound management products should be minimally adhesive to skin to facilitate non-traumatic wound dressing removal and minimise damage to newly formed skin layer. However, under the Acticoat™ series, only Acticoat™ Absorbent is minimally adhesive as its main function is to absorb exudate which allows gel formation and prevents attachment to newly formed skin layer. However, not all wounds release sufficient amount of exudate.

Other criteria that wound therapy agents should preferably possess is the ability to attract growth factors to the site of injury to spur cell regeneration, being flexible to facilitate application of therapeutics over awkward positions, having water proof feature and can be synthesised at low cost.

There is therefore a need to provide biomaterials that overcome, or at least ameliorate, one or more of the disadvantages described above.

### Summary

The invention is defined by the claims. Any subject-matter falling outside the scope of the claims is provided for information purposes only.

The invention concerns a nanoparticle-containing hydrogel comprising:
metal nanoparticles selected from the group consisting of Ag nanoparticles, Cu nanoparticles, CuO nanoparticles, Fe₂O₃ nanoparticles, Pt nanoparticles, Pd nanoparticles and
combinations thereof; and
at least one peptide having the general formula:

   Z-(X)ₐ-(Y)_{b}-Z'_{c}

   wherein
   Z is an N-terminal protecting group;
   X is, at each occurrence, independently selected from the group consisting of aliphatic amino acids;
   Y is, at each occurrence, independently selected from the group consisting of polar amino acids, wherein said polar amino acids have a polar group which is independently selected from a hydroxyl, an ether, an imido, an amido, an ester, an amino, a guanidino, a seleno, and a telluro group;
   Z' is a C-terminal protecting group;
   a is an integer selected from 1 to 6, preferably 1 to 5;
   b is 1 or 2, preferably 1; and
   c is 0 or 1.

Advantageously, the peptide(s) may be able to self-assemble in water or physiologically relevant buffers to form hydrogels.

Further advantageously, the hydrogels may display superior mechanical strength. The metal nanoparticles may stabilize the fibrous structure of the hydrogel, therefore requiring a reduced concentration of peptide(s), which are considerably more expensive. Therefore advantageously, the disclosed hydrogels may be cost-effective.

Another object of the invention concerns an in situ method of producing the above nanoparticle-containing hydrogel, said method comprising the steps of
(a) dissolving at least one peptide as defined above in an aqueous solution;
(b) adding a precursor of metal nanoparticles, preferably a metal salt solution, wherein the metal salt is selected from AgNO₃, CuSO₄, CuSO₄*5H₂O, Cu(NO₃)₂ and its hydrates, Cu(OAc)₂ and its hydrates, FeCl₃ and its hydrates, FeCl₂ and its hydrates, K₂PtCl₄, PdCl₂, H₂PdCl₄ and Pd(NO₃)₂;
(c) gel formation to obtain a peptide hydrogel;
(d) irradiating the peptide hydrogel resulting from step (c); and
(e) obtaining a nanoparticle-containing hydrogel.

Another object of the invention concerns the use of the above nanoparticle-containing hydrogel
- for bioimaging (by utilizing the fluorescence of the metal nanoparticles);
- for application where the hydrogel is used as a (dried) membrane, such as filtration; or
- in a cosmetic composition.

Another object of the invention concerns a bioimaging device comprising the above nanoparticle-containing hydrogel for in vitro and/or in vivo use, preferably for oral application, for injection and/or for topical application.

Finally, another object of the invention concerns the above nanoparticle-containing hydrogel for use in treating an infectious disease, in preventing bacterial attachment and/ or formation of microbial biofilm, in combination with medical devices, such as stents, prostheses, catheters, dental implants, orthopedical devices, or orthopedical implants, or in a pharmaceutical composition.

Advantageously, the claimed hydrogels may not require additional chemical modification to display antibacterial and/or antimicrobial properties.

According to the present disclosure this object is solved by a device for metal delivery, preferably sustained or controlled release metal delivery, comprising the claimed nanoparticle-containing hydrogel.

Advantageously, the claimed hydrogels may display sustained release of the metal nanoparticles over a long period, for example, more than 10 days.

Further advantageously, the claimed hydrogels may be able to fully release the metal nanoparticles, even while using a low concentration of metal salt precursor.

Also advantageously, the claimed hydrogels may only require low concentrations of metal salt precursor, which may be toxic in high amounts.

According to the present disclosure this object is solved by an antimicrobial agent comprising the claimed nanoparticle-containing hydrogel.

According to the present disclosure this object is solved by a wound dressing or wound healing agent comprising the claimed nanoparticle-containing hydrogel, which is preferably used to prevent microbial infection and/or formation of microbial biofilm.

Advantageously, the claimed hydrogels may be able to prevent bacterial attachment, and therefore prevent the formation of biofilm.

Advantageously, the claimed hydrogels may be able to entrap up to 99.9% water. Therefore advantageously, the moisture from the claimed hydrogels may prevent unwanted adhesion when applied to wound sites, facilitating non-traumatic removal of the dressing. The claimed hydrogels offer great potential for application in wound healing, in particular after surgery, in treatment of chronic and large surface wounds, like diabetic ulcer or severe burns, due to its potential to reduce inflammation and its ease of application.

According to the present disclosure this object is solved by a pharmaceutical or cosmetic composition comprising the claimed nanoparticle-containing hydrogel.

According to the present disclosure this object is solved by the claimed hydrogel for use in a method of treatment of an infectious disease, said method comprising the step of administering an effective amount of the claimed nanoparticle-containing hydrogel or a disclosed pharmaceutical composition to a person or subject in need thereof, wherein, preferably, said infectious disease is caused by Gram-positive bacteria, Gram-negative bacteria, yeast or fungi.

According to the present disclosure this object is solved by the claimed hydrogel for use in a method of treatment of a wound and for wound healing, said method comprising the step of applying an effective amount of the claimed nanoparticle-containing hydrogel or a disclosed pharmaceutical composition to a wound.

Advantageously, the claimed hydrogels may be flexible and therefore may be able to be applied across awkward or non-flat positions on the skin.

According to the present disclosure this object is solved by the claimed nanoparticle-containing hydrogel for use in the treatment of a wound and/or for wound healing.

According to the present disclosure this object is solved by a kit, comprising
(i) at least one peptide as defined herein, optionally, in an aqueous solution;
(ii) a precursor of metal nanoparticles as disclosed above, preferably a metal salt solution (such as AgNO₃);
(iii) instructions for gel formation to obtain a peptide hydrogel and for irradiating the peptide hydrogel resulting therefrom for obtaining a nanoparticle-containing hydrogel;
(iv) optionally, excipients.

According to the present disclosure this object is solved by a kit, comprising
(i) a hydrogel obtained from at least one peptide as defined herein, preferably in a container to be protected from radiation;
(ii) a precursor of metal nanoparticles as disclosed above, preferably a metal salt solution (such as AgNO₃);
(iii) instructions for irradiating the peptide hydrogel for obtaining a nanoparticle-containing hydrogel;
(iv) optionally, excipients.

### Definitions

The following are some definitions that may be helpful in understanding the description of the present invention. These are intended as general definitions and should in no way limit the scope of the present invention to those terms alone, but are put forth for a better understanding of the following description.

Unless the context requires otherwise or specifically stated to the contrary, integers, steps, or elements of the invention recited herein as singular integers, steps or elements clearly encompass both singular and plural forms of the recited integers, steps or elements.

Throughout this specification, unless the context requires otherwise, the word "comprise", or variations such as "comprises" or "comprising", will be understood to imply the inclusion of a stated step or element or integer or group of steps or elements or integers, but not the exclusion of any other step or element or integer or group of elements or integers. Thus, in the context of this specification, the term "comprising" means "including principally, but not necessarily solely".

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations or any two or more of said steps or features.

As used herein, the term "amino acid" refers to a compound or molecule having at least one primary, secondary, tertiary or quaternary amino group, and at least one acid group, wherein the acid group may be a carboxylic, sulfonic, or phosphonic acid, or mixtures thereof. The amino groups may be "alpha", "beta", "gamma" to "omega" with respect to the acid group(s). The backbone of the "amino acid" may be substituted with one or more groups selected from halogen, hydroxy, guanido, heterocyclic groups. Thus the term "amino acids" also includes within its scope glycine, homoallylglycine, homopropargylglycine, alanine, valine, leucine, isoleucine, norleucine, proline, phenylalanine, tryptophane, serine, threonine, tyrosine, asparagine, glutamine, asparte, glutamine, lysine, taurine, betaine, N-methylalanine etc. (L) and (D) forms of amino acids are also included in the scope of this invention.

As used herein, the term "amino acid derivatives" refers to various derivatives of the above-mentioned amino acids. Examples thereof include an unusual amino acid, a non-natural amino acid, an amino alcohol, or an amino acid in which an amino acid side chain such as the terminal carbonyl group, the terminal amino group, is replaced with any one of various substituents. Examples of the substituents include an alkyl group, an acyl group, a hydroxyl group, an amino group, an alkylamino group, a nitro group, a sulfonyl group, various protection groups, and the like. For example, Val-NH₂: valinamide, Val-ol: valinol (2-amino-3-methyl-1-butanol) and the like are included. Amino acid derivatives are not according to the invention.

As used herein, the terms "polar amino acid" or "polar amino acid derivatives" refer to amino acids having one or more polar groups. The polar group may be hydroxyl, ether, carboxyl, imido, amido, ester, amino, seleno, and/or telluro. For example, the term "polar amino acid" or "polar amino acid derivatives" includes, but is not limited to aspartic acid (Asp, D), asparagine (Asn, N), glutamic acid (Glu, E), glutamine (Gln, Q), 5-N-ethyl-glutamine (theanine), citrulline, serine (Ser, S), homoserine,-ornithine (Orn), threonine (Thr, T), L-Dopa, tryptophan (Trp, W), thyroxine, allo-threonine, lysine (Lys, K), 2,4-diaminobutyric acid (Dab), 2,3-diaminopropionic acid (Dap), N(6)-carboxymethyllysine, and tyrosine (Tyr, Y). Polar amino acids with a carboxyl polar group are not according to the invention. Polar amino acid derivatives are also not according to the invention.

As used herein, the term "alkyl" includes within its meaning monovalent ("alkyl") and divalent ("alkylene") straight chain or branched chain saturated aliphatic groups having from 1 to 12 carbon atoms, eg, 1, 2, 3, 4, 5, 6, 7, 8, 9, 10, 11 or 12 carbon atoms. For example, the term alkyl includes, but is not limited to, methyl, ethyl, 1-propyl, isopropyl, 1-butyl, 2-butyl, isobutyl, tert-butyl, amyl, 1,2-dimethylpropyl, 1,1-dimethylpropyl, pentyl, isopentyl, hexyl, 4-methylpentyl, 1-methylpentyl, 2-methylpentyl, 3-methylpentyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, 2-ethylpentyl, 3-ethylpentyl, heptyl, 1-methylhexyl, 2,2-dimethylpentyl, 3,3-dimethylpentyl, 4,4-dimethylpentyl, 1,2-dimethylpentyl, 1,3-dimethylpentyl, 1,4-dimethylpentyl, 1,2,3-trimethylbutyl, 1,1,2-trimethylbutyl, 1,1,3-trimethylbutyl, 5-methylheptyl, 1-methylheptyl, octyl, nonyl, decyl, undecyl, dodecyl and the like. Alkyl groups may be optionally substituted.

As used herein, the term "alkene" includes within its meaning monovalent ("alkene" or "alkenyl") and divalent ("alkenylene") straight or branched chain unsaturated aliphatic hydrocarbon groups having from 2 to 10 carbon atoms, eg, 2, 3, 4, 5, 6, 7, 8, 9, or 10 carbon atoms and having at least one double bond, of either *E*, *Z*, *cis* or *trans* stereochemistry where applicable, anywhere in the alkyl chain. Examples of alkenyl groups include but are not limited to ethenyl, vinyl, allyl, 1-methylvinyl, 1-propenyl, 2-propenyl, 2-methyl-1-propenyl, 2-methyl-1-propenyl, 1-butenyl, 2-butenyl, 3-butentyl, 1,3-butadienyl, 1-pentenyl, 2-pententyl, 3-pentenyl, 4-pentenyl, 1,3-pentadienyl, 2,4-pentadienyl, 1,4-pentadienyl, 3-methyl-2-butenyl, 1-hexenyl, 2-hexenyl, 3-hexenyl, 1,3-hexadienyl, 1,4-hexadienyl, 2-methylpentenyl, 1-heptenyl, 2-heptentyl, 3-heptenyl, 1-octenyl, 1-nonenyl, 1-decenyl, and the like.

As used herein, the term "alkyne" includes within its meaning monovalent ("alkyne" or "alkynyl") and divalent ("alkynylene") straight or branched chain unsaturated aliphatic hydrocarbon groups having from 2 to 10 carbon atoms and having at least one triple bond anywhere in the carbon chain. Examples of alkynyl groups include but are not limited to ethynyl, 1-propynyl, 1-butynyl, 2-butynyl, 1-methyl-2-butynyl, 3-methyl-1-butynyl, 1-pentynyl, 1-hexynyl, methylpentynyl, 1-heptynyl, 2-heptynyl, 1-octynyl, 2-octynyl, 1-nonyl, 1-decynyl, and the like.

As used herein, the term "amine" or "amino" refers to groups of the form - NRₐR_{b} wherein Rₐ and R_{b} are individually selected from the group including but not limited to hydrogen, optionally substituted alkyl, optionally substituted alkenyl, optionally substituted alkynyl, and optionally substituted aryl groups.

As used herein, the term "amide" refers to compounds or moieties that contain a nitrogen atom bound to the carbon of a carbonyl. The term includes "alkaminocarbonyl" or "alkylaminocarbonyl" groups which include alkyl, alkenyl, aryl or alkynyl groups bound to an amino group bound to a carbonyl group. It includes "arylaminocarbonyl" and "arylcarbonylamino" groups which include aryl or heteroaryl moieties bound to an amino group which is bound to the carbon of a carbonyl. The terms "alkylaminocarbonyl," "alkenylaminocarbonyl," "alkynylaminocarbonyl," "arylaminocarbonyl," "alkylcarbonylamino," "alkenylcarbonylamino," "alkynylcarbonylamino," and "arylcarbonylamino" are included in term "amide." Amides z'also include urea groups (aminocarbonylamino) and carbamates (oxycarbonylamino).

As used herein, the term "imine" includes within its meaning the reaction product of an amine or ammonia and an aldehyde or ketone. This reaction results in a molecule with at least one C=N group.

As used herein, the term "urea analog" refers to the various derivatives of urea or carbamid such as benzoyl carbamid, acetyl carbamid. And wherein we have used the term furfural, we wish it to be understood as including its homologues, derivatives and substitution products.

As used herein, the term "aryl", or variants such as "aromatic group" or "arylene" refers to monovalent ("aryl") and divalent ("arylene") single, polynuclear, conjugated and fused residues of aromatic hydrocarbons having from 6 to 10 carbon atoms. Such groups include, for example, phenyl, biphenyl, naphthyl, phenanthrenyl, and the like. Aryl groups may be optionally substituted.

As used herein, the term "optionally substituted" means the group to which this term refers may be unsubstituted, or may be substituted with one or more groups independently selected from alkyl, alkenyl, alkynyl, cycloalkyl, cycloalkenyl, heterocycloalkyl, halo, carboxyl, haloalkyl, haloalkynyl, hydroxyl, alkoxy, alkenyloxy, haloalkoxy, haloalkenyloxy, nitro, amino, nitroalkyl, nitroalkenyl, nitroalkynyl, nitroheterocyclyl, alkylamino, dialkylamino, alkenylamine, alkynylamino, acyl, alkenoyl, alkynoyl, acylamino, diacylamino, acyloxy, alkylsulfonyloxy, heterocycloxy, heterocycloamino, haloheterocycloalkyl, alkylsulfenyl, alkylcarbonyloxy, phosphorus-containing groups such as phosphono and phosphinyl, aryl, heteroaryl, alkylaryl, alkylheteroaryl, cyano, cyanate, isocyanate, -C(O)NH(alkyl), and -C(O)N(alkyl)₂.

As used herein, the term "aliphatic" refers to an organic compound or radical characterized by a straight chain or branched chain structure, or closed ring structure, any of which may contain saturated carbon bonds, and optionally, one or more unconjugated carbon-carbon unsaturated bonds, such as a carbon-carbon double bond. For the purposes of this invention, the term "aliphatic" also includes "alicyclic" compounds defined hereinafter. The aliphatic groups may have from 1 to 24 carbon atoms eg, 1, 2, 3, 4, 5, 6, 7, 8, 9, 10, 11, 12, 13, 14, 15, 16, 17, 18, 19, 20, 21, 22, 23, 24 carbon atoms.

As used herein, the term "metal nanoparticles" refers to nanoparticles comprising or consisting of metal and/or metal oxides. The term "metal nanoparticles" can also refer to heterometallic nanoparticles, heterometallic oxide nanoparticles or combinations thereof.

As used herein, the term "heterometallic nanoparticle" refers to a nanoparticle consisting or comprising of one metal and one metalloid atom in the same compound.

As used herein, the term "heterometallic oxide nanoparticle" refers to a nanoparticle consisting or comprising of one metal oxide and one metalloid oxide atom in the same compound.

The nanoparticles of the invention are Ag nanoparticles, Cu nanoparticles, CuO nanoparticles, Fe₂O₃ nanoparticles, Pt nanoparticles, Pd nanoparticles and combinations thereof.

As used herein, the term "pharmaceutically acceptable salt" includes, for example, the hydrochloride, hydrobromide, hydroiodide, sulfate, phosphate, acetate, propionate, lactate, maleate, malate, succinate, and tartrate salts.

As used herein, the term "pharmaceutically acceptable carrier" is intended to include solvents, dispersion media, coatings, anti-bacterial and anti-fungal agents, isotonic and absorption delaying agents, and the like. The use of such media and agents for pharmaceutically active substances is well known in the art. Except insofar as any conventional media or agent is incompatible with the compound, use thereof in the therapeutic compositions and methods of treatment and prophylaxis is contemplated. Supplementary active compounds may also be incorporated into the compositions according to the present invention. It is especially advantageous to formulate parenteral compositions in dosage unit form for ease of administration and uniformity of dosage. "Dosage unit form" as used herein refers to physically discrete units suited as unitary dosages for the individual to be treated; each unit containing a predetermined quantity of compound(s) is calculated to produce the desired therapeutic effect in association with the required pharmaceutical carrier. The compound(s) may be formulated for convenient and effective administration in effective amounts with a suitable pharmaceutically acceptable carrier in an acceptable dosage unit. In the case of compositions containing supplementary active ingredients, the dosages are determined by reference to the usual dose and manner of administration of the said ingredients.

The present invention includes within its scope all isomeric forms of the compounds disclosed herein, including all diastereomeric isomers, racemates and enantiomers, for example, *E, Z, cis, trans,* (R), (S), (L), (D), (+), and/or (-) forms of the compounds, as appropriate in each case.

In the context of this invention the term "administering" and variations of that term including "administer" and "administration", includes contacting, applying, delivering or providing a compound or composition of the invention to an organism, or a surface by any appropriate means.

In the context of this specification, the term "treatment", refers to any and all uses which remedy a disease state or symptoms, prevent the establishment of disease, or otherwise prevent, hinder, retard, or reverse the progression of disease or other undesirable symptoms in any way whatsoever.

In the context of this specification the terms "therapeutically effective amount" and "diagnostically effective amount", include within their meaning a sufficient but non-toxic amount of a compound or composition of the invention to provide the desired therapeutic or diagnostic effect. The exact amount required will vary from subject to subject depending on factors such as the species being treated, the age and general condition of the subject, the severity of the condition being treated, the particular agent being administered, the mode of administration, and so forth. Thus, it is not possible to specify an exact "effective amount". However, for any given case, an appropriate "effective amount" may be determined by one of ordinary skill in the art using only routine experimentation.

The word "substantially" does not exclude "completely" e.g. a composition which is "substantially free" from Y may be completely free from Y. Where necessary, the word "substantially" may be omitted from the definition of the invention.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1% of the stated value, and even more typically +/- 0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Certain embodiments may also be described broadly and generically herein. Each of the narrower species and subgeneric groupings falling within the generic disclosure also form part of the disclosure. This includes the generic description of the embodiments with a proviso or negative limitation removing any subject matter from the genus, regardless of whether or not the excised material is specifically recited herein.

### Detailed Disclosure of Embodiments

Exemplary, non-limiting embodiments of the disclosed nanoparticle-containing hydrogel will now be disclosed.

The present disclosure provides a nanoparticle-containing hydrogel.

Said nanoparticle-containing hydrogel comprises:
metal nanoparticles, and
at least one peptide having the general formula:

   ***Z-*(X)ₐ-(Y)_{b}-*Z'_{c}***

   wherein
   *Z* is an N-terminal protecting group;
   X is, at each occurrence, independently selected from the group consisting of aliphatic amino acids and aliphatic amino acid derivatives;
   **Y** is, at each occurrence, independently selected from the group consisting of polar amino acids and polar amino acid derivatives;
   ***Z**'* is a C-terminal protecting group;
   **a** is an integer selected from 1 to 6, preferably 1 to 5;
   **b** is 0, 1 or 2, preferably 1; and
   **c** is 0 or 1.

The metal nanoparticles may be metal nanoparticles, metal oxide nanoparticles, and combinations thereof. The nanoparticles are preferably selected from silver (Ag) nanoparticles, gold (Au) nanoparticles, copper (Cu) nanoparticles, copper(II) oxide (CuO) nanoparticles, iron(III) oxide (Fe₂O₃) nanoparticles, platinum (Pt) nanoparticles, palladium (Pd) nanoparticles or combinations thereof. The metal nanoparticles may be heterometallic nanoparticles, heterometallic oxide nanoparticles or combinations thereof.

In particular, the nanoparticle-containing hydrogel according to the invention comprises
metal nanoparticles selected from the group consisting of Ag nanoparticles, Cu nanoparticles, CuO nanoparticles, Fe₂O₃ nanoparticles, Pt nanoparticles, Pd nanoparticles and combinations thereof; and
at least one peptide having the general formula:

Z-(X)ₐ-(Y)_{b}-Z'_{c}

wherein
Z is an N-terminal protecting group;
X is, at each occurrence, independently selected from the group consisting of aliphatic amino acids;
Y is, at each occurrence, independently selected from the group consisting of polar amino acids, wherein said polar amino acids have a polar group which is independently selected from a hydroxyl, an ether, an imido, an amido, an ester, an amino, a guanidino, a seleno, and a telluro group;
Z' is a C-terminal protecting group;
a is an integer selected from 1 to 6, preferably 1 to 5;
b is 1 or 2, preferably 1; and
c is 0 or 1.

The metal nanoparticles may have a uniform size distribution in the hydrogel, preferably having a particle size of less than about 1 µm, more preferably about 10 to about 20 nm.

The metal nanoparticles may have a particle size of less than about 1 µm, less than about 0.8 µm, less than about 0.7 µm, less than about 0.6 µm, less than about 0.5 µm, less than about 0.4 µm, less than about 0.3µm, less than about 0.2 µm, less than about 0.1 µm, less than about 100 nm, less than about 80 nm, less than about 60 nm, less than about 40 nm, less than about 20 nm, less than about 10 nm, about 10 nm, about 20nm, from about 10 to about 100 nm, form about 10 to about 50 nm, from about 10 to about 20 nm.

The aliphatic amino acids or aliphatic amino acid derivatives may be either D-amino acids or L-amino acids. Aliphatic amino acid derivatives are not according to the invention.

The aliphatic amino acid may be independently selected from alanine (Ala, A), homoallylglycine homopropargylglycine, isoleucine (Ile, I), norleucine, leucine (Leu, L), valine (Val, V) and glycine (Gly, G), preferably from the group consisting of alanine (Ala, A), isoleucine (Ile, I), leucine (Leu, L), valine (Val, V) and glycine (Gly, G).

The aliphatic amino acids or aliphatic amino acid derivatives, all or a portion thereof, may be arranged in an order of decreasing amino acid size in the direction from N- to C-terminus, wherein the size of the aliphatic amino acids is defined as I = L>V>A>G. Aliphatic amino acid derivatives are not according to the invention.

The aliphatic amino acids or aliphatic amino acid derivatives may be arranged in an order of decreasing amino acid size with a sequence selected from LIVAG (SEQ ID NO: 1), ILVAG (SEQ ID NO: 2), LIVAA (SEQ ID NO: 3), IVAG (SEQ ID NO: 5), LIVA (SEQ ID NO: 6), LIVG (SEQ ID NO: 7), IVA (SEQ ID NO: 23) and IV (SEQ ID NO: 24), wherein, optionally, there is an A preceding such sequence at the N-terminus. See also the following table 1. In this table, the peptide with SEQ ID NO: 4 is not characterized by the amino acids being arranged in an order of decreasing size. Optionally, there may be an A preceding such sequence at the N-terminus, such as AIVAG. Aliphatic amino acid derivatives are not according to the invention.

**Table 1**

| **SEQ ID NO.** | **Sequence** |
|---|---|
| 1 | LIVAG |
| 2 | ILVAG |
| 3 | LIVAA |
| 4 | LAVAG |
| 5 | IVAG |
| 6 | LIVA |
| 7 | LIVG |
| 23 | IVA |
| 24 | IV |

The polar amino acids or polar amino acid derivatives may be either D-amino acids or L-amino acids. Polar amino acid derivatives are not according to the invention.

The polar amino acids or polar amino acid derivatives have a polar group which is independently selected from a hydroxyl, an ether, a carboxyl, an imido, an amido, an ester, an amino, a guanidino, a seleno, and a telluro group. Polar amino acids with a carboxyl polar group are not according to the invention. Polar amino acid derivatives are also not according to the invention.

The polar amino acids or polar amino acid derivatives may be independently selected from aspartic acid (Asp, D), asparagine (Asn, N), glutamic acid (Glu, E), glutamine (Gln, Q), 5-N-ethyl-glutamine (theanine), citrulline, serine (Ser, S), homoserine, ornithine (Orn), threonine (Thr, T), methionine, L-Dopa, tryptophan (Trp, W), thyroxine, allo-threonine, lysine (Lys, K), 2,4-diaminobutyric acid (Dab), 2,3-diaminopropionic acid (Dap), N(6)-carboxymethyllysine, or tyrosine (Tyr, Y), preferably selected from the group consisting of aspartic acid, asparagine, glutamic acid, glutamine, serine, threonine, methionine, lysine, ornithine (Orn), 2,4-diaminobutyric acid (Dab), and 2,3-diaminopropionic acid (Dap).
In the peptide having the general formula: Z-(X)ₐ-(Y)_{b}-Z'_{c}, the moiety -(X)ₐ-(Y)_{b}- may be selected from LIVAGD (SEQ ID NO: 8), ILVAGD (SEQ ID NO: 9), LIVAAD (SEQ ID NO: 10), LAVAGD (SEQ ID NO: 11), AIVAGD (SEQ ID NO: 12), LIVAGE (SEQ ID NO: 13), LIVAGK (SEQ ID NO: 14), ILVAGK (SEQ ID NO. 15), LIVAGT (SEQ ID NO: 16), AIVAGT (SEQ ID NO: 17), LIVAD (SEQ ID NO: 18), LIVGD (SEQ ID NO: 19), IVAD (SEQ ID NO: 20), IIID (SEQ ID NO: 21), IIIK (SEQ ID NO: 22), IVD (SEQ ID NO: 25), IID (SEQ ID NO: 26), LVE (SEQ ID NO: 27), IVE (SEQ ID NO: 28), LVD (SEQ ID NO: 29), VIE (SEQ ID NO: 30), VID (SEQ ID NO: 31), VLD (SEQ ID NO: 32), VLE (SEQ ID NO: 33), LLE (SEQ ID NO: 34), LLD (SEQ ID NO: 35), IIE (SEQ ID NO: 36), ID (SEQ ID NO: 37), IE (SEQ ID NO: 38) and IY (SEQ ID NO: 39). They may be selected from the following table 2:

**Table 2**

| **SEQ ID NO.** | **Sequence** | **SEQ ID NO.** | **Sequence** |
|---|---|---|---|
| 8 | LIVAGD | 25 | IVD |
| 9 | ILVAGD | 26 | IID |
| 10 | LIVAAD | 27 | LVE |
| 11 | LAVAGD | 28 | IVE |
| 12 | AIVAGD | 29 | LVD |
| 13 | LIVAGE | 30 | VIE |
| 14 | LIVAGK | 31 | VID |
| 15 | ILVAGK | 32 | VLD |
| 16 | LIVAGT | 33 | VLE |
| 17 | AIVAGT | 34 | LLE |
| 18 | LIVAD | 35 | LLD |
| 19 | LIVGD | 36 | IIE |
| 20 | IVAD | 37 | ID |
| 21 | IIID | 38 | IE |
| 22 | IIIK | 39 | IY |

Polar amino acids with a carboxyl polar group are not according to the invention. Polar amino acid derivatives are also not according to the invention. The C-terminal amino acid of the peptide may be selected from lysine (K), an ornithine (Orn), a 2,4-diaminobutyric acid (Dab), or a 2,3-diaminopropionic acid (Dap).

In the peptide having the general formula: Z-(X)ₐ-(Y)_{b}-Z'_{c}, the N-terminal protecting group Z may have the general formula -C(O)-R, wherein R is selected from the group consisting of H, unsubstituted or substituted alkyls, and unsubstituted or substituted aryls. R may be methyl, ethyl, propyl, isopropyl, butyl or isobutyl.

Z may be an acetyl group.

Z may be a peptidomimetic molecule, including natural and synthetic amino acid derivatives, wherein the N-terminus of said peptidomimetic molecule may be modified with a functional group selected from the group consisting of carboxylic acid, amide, alcohol, aldehyde, amine, imine, nitrile, an urea analog, phosphate, carbonate, sulfate, nitrate, maleimide, vinyl sulfone, azide, alkyne, alkene, carbohydrate, imide, peroxide, ester, aryl, ketone, sulphite, nitrite, phosphonate, and silane.

In the peptide having the general formula: Z-(X)ₐ-(Y)_{b}-Z'_{c}, the C-terminal protecting group Z' may be an amide group. Z' may be of the formula -CONHR or - CONRR', with R and R' being selected from the group consisting of H, unsubstituted or substituted alkyls, and unsubstituted or substituted aryls. R and R' may independently be methyl, ethyl, propyl, isopropyl, butyl or isobutyl.

Z' may be an ester group. Z' may be of the formula -CO₂R, with R being selected from the group consisting of H, unsubstituted or substituted alkyls, and unsubstituted or substituted aryls. R may be methyl, ethyl, propyl, isopropyl, butyl or isobutyl.

Z' may be a peptidomimetic molecule, including natural and synthetic amino acid derivatives, wherein the C-terminus of said peptidomimetic molecule may be modified with a functional group selected from the group consisting of carboxylic acid, amide, alcohol, aldehyde, amine, imine, nitrile, an urea analog, phosphate, carbonate, sulfate, nitrate, maleimide, vinyl sulfone, azide, alkyne, alkene, carbohydrate, imide, peroxide, ester, aryl, ketone, sulphite, nitrite, phosphonate, and silane.

In the peptide having the general formula according to the first aspect: Z-(X)ₐ-(Y)_{b}-Z'_{c}, a may be an integer selected from 1 to 5, preferably 2 to 5. a may be 1, 2, 3, 4, or 5. The aliphatic amino acids and aliphatic amino acid derivatives of X may exhibit an overall decrease in hydrophobicity from the N-terminus to the C-terminus of said peptide. Aliphatic amino acid derivatives are not according to the invention.

The peptide may be present at a concentration in the range of from about 0.1 % to about 30.0 % (w/w) preferably 0.1 % to 20 % (w/w), more preferably 0.1 % to 10 % (w/w), more preferably 0.1 % to 5 % (w/w), even more preferably 0.1 % to 3 % (w/w), with respect to the total weight of said nanoparticle-containing hydrogel.

The peptide may be present at a concentration in the range of from about 0.5 % to about 30.0 % (w/w), from about 1% to about 30 % (w/w), from about 2 % to about 30 % (w/w), from about 3 % to about 30 % (w/w), from about 4 % to about 30 % (w/w), from about 5 % to about 30 % (w/w), from about 6 % to about 30 % (w/w), from about 7 % to about 30 % (w/w), from about 8 % to about 30 % (w/w), from about 9 % to about 30 % (w/w), from about 10 % to about 30 % (w/w), from about 11 % to about 30 % (w/w), from about 12 % to about 30 % (w/w), from about 13 % to about 30 % (w/w), from about 14 % to about 30 % (w/w), from about 15 % to about 30 % (w/w), from about 16 % to about 30 % (w/w), from about 17 % to about 30 % (w/w), from about 18 % to about 30 % (w/w), from about 19 % to about 30 % (w/w), from about 20 % to about 30 % (w/w), from about 21 % to about 30 % (w/w), from about 22 % to about 30 % (w/w), from about 23 % to about 30 % (w/w), from about 24 % to about 30 % (w/w), from about 25 % to about 30 % (w/w), from about 26 % to about 30 % (w/w), from about 27 % to about 30 % (w/w), from about 28 % to about 30 % (w/w), from about 29 % to about 30 % (w/w), from about 0.1 % to about 29 % (w/w), from about 0.1 % to about 28 % (w/w), from about 0.1 % to about 27 % (w/w), from about 0.1 % to about 26 % (w/w), from about 0.1 % to about 25 % (w/w), from about 0.1 % to about 24 % (w/w), from about 0.1 % to about 23 % (w/w), from about 0.1 % to about 22 % (w/w), from about 0.1 % to about 21 % (w/w), from about 0.1 % to about 20 % (w/w), from about 0.1 % to about 19 % (w/w), from about 0.1 % to about 18 % (w/w), from about 0.1 % to about 17 % (w/w), from about 0.1 % to about 16 % (w/w), from about 0.1 % to about 15 % (w/w), from about 0.1 % to about 14 % (w/w), from about 0.1 % to about 13 % (w/w), from about 0.1 % to about 12 % (w/w), from about 0.1 % to about 11 % (w/w), from about 0.1 % to about 10 % (w/w), from about 0.1 % to about 9 % (w/w), from about 0.1 % to about 8 % (w/w), from about 0.1 % to about 7 % (w/w), from about 0.1 % to about 6 % (w/w), from about 0.1 % to about 5 % (w/w), from about 0.1 % to about 4 % (w/w), from about 0.1 % to about 3 % (w/w), from about 0.1 % to about 2 % (w/w), from about 0.1 % to about 1 % (w/w), or from about 0.1 % to about 0.5 % (w/w). The peptide may be present at a concentration of about 0.1 % (w/w), about 0.5 % (w/w), about 1 % (w/w), about 2 % (w/w), about 3 % (w/w), about 4 % (w/w), about 5 % (w/w), about 6 % (w/w), about 7 % (w/w), about 8 % (w/w), about 9 % (w/w), about 10 % (w/w), about 11 % (w/w), about 12 % (w/w), about 13 % (w/w), about 14 % (w/w), about 15 % (w/w), about 16 % (w/w), about 17 % (w/w), about 18 % (w/w), about 19 % (w/w), about 20 % (w/w), about 21 % (w/w), about 22 % (w/w), about 23 % (w/w), about 24 % (w/w), about 25 % (w/w), about 26 % (w/w), about 27 % (w/w), about 28 % (w/w), about 29 % (w/w), or about 30 % (w/w).

The nanoparticle-containing hydrogel may further comprise at least one additional substance. The additional substance may be encapsulated by the hydrogel, immobilized in the bulk phase of the hydrogel, or conjugated to the peptide.

The additional substance may be selected from the group consisting of dyes, pigments, quantum dot nanoparticles and semiconductor nanoparticles.

The present disclosure provides an *in situ* method of producing a disclosed nanoparticle-containing hydrogel.

Said method comprises the steps of
(a) dissolving at least one disclosed peptide in an aqueous solution;
(b) adding a precursor of metal nanoparticles, preferably a metal salt solution;
(c) gel formation to obtain a peptide hydrogel;
(d) irradiating the peptide hydrogel resulting from step (c); and
(e) obtaining a nanoparticle-containing hydrogel.

The metal salt may be selected from the group consisting of AgNO₃, AuCl₃, HAuCl₄, CuSO₄, CuSO₄.5H₂O, Cu(NO₃)₂ and its hydrates, Cu(OAc)₂ and its hydrates, FeCl₃ and its hydrates, FeCl₂ and its hydrates (and all iron chloride hydrates), K₂PtCl₄, PdCl₂, H₂PdCl₄ and Pd(NO₃)₂.
In particular, the invention concerns an *in situ* method of producing the nanoparticle-containing hydrogel according to the invention comprising the steps of
(a) dissolving at least one peptide as defined above in an aqueous solution;
(b) adding a precursor of metal nanoparticles, preferably a metal salt solution, wherein the metal salt is selected from AgNO₃, CuSO₄, CuSO₄^{∗}5H₂O, Cu(NO₃)₂ and its hydrates, Cu(OAc)₂ and its hydrates, FeCl₃ and its hydrates, FeCl₂ and its hydrates, K₂PtCl₄, PdCl₂, H₂PdCl₄ and Pd(NO₃)₂;
(c) gel formation to obtain a peptide hydrogel;
(d) irradiating the peptide hydrogel resulting from step (c); and
(e) obtaining a nanoparticle-containing hydrogel.

The concentration of the precursor may be in the range from about 0.001 M to about 1 M, or from about 1 mM to about 1000 mM, preferably 1 to 100mM, even more preferred 10 to 50mM.

The concentration of the precursor may be in the range from about 50 mM to about 1000 mM, from about 100 mM to about 1000 mM, from about 150 mM to about 1000 mM, from about 200 mM to about 1000 mM, from about 250 mM to about 1000 mM, from about 300 mM to about 1000 mM, from about 350 mM to about 1000 mM, from about 400 mM to about 1000 mM, from about 450 mM to about 1000 mM, from about 500 mM to about 1000 mM, from about 550 mM to about 1000 mM, from about 600 mM to about 1000 mM, from about 650 mM to about 1000 mM, from about 700 mM to about 1000 mM, from about 750 mM to about 1000 mM, from about 800 mM to about 1000 mM, from about 850 mM to about 1000 mM, from about 900 mM to about 1000 mM, from about 950 mM to about 1000 mM, from about 1 mM to about 950 mM, from about 1 mM to about 900 mM, from about 1 mM to about 850 mM, from about 1 mM to about 800 mM, from about 1 mM to about 750 mM, from about 1 mM to about 700 mM, from about 1 mM to about 650 mM, from about 1 mM to about 600 mM, from about 1 mM to about 550 mM, from about 1 mM to about 500 mM, from about 1 mM to about 450 mM, from about 1 mM to about 400 mM, from about 1 mM to about 350 mM, from about 1 mM to about 300 mM, from about 1 mM to about 250 mM, from about 1 mM to about 200 mM, from about 1 mM to about 150 mM, from about 1 mM to about 100 mM, or from about 1 mM to about 50 mM. The concentration of the precursor may be about 1 mM, about 50 mM, about 100 mM, about 150 mM, about 200 mM, about 250 mM, about 300 mM, about 350 mM, about 400 mM, about 450 mM, about 500 mM, about 550 mM, about 600 mM, about 650 mM, about 700 mM, about 750 mM, about 800 mM, about 850 mM, about 900 mM, about 950 mM, or about 1000 mM.

Step (b) of the disclosed synthetic method may be carried out in water or in chloride and phosphate free buffer, preferably Tris buffer, either at physiological or at a basic pH, such as pH of about 7.4 or about 8.5, or from about pH 7 to about pH 9.

The pH may be about pH 7.2, about pH 7.4, about pH 7.6, about pH 7.8, about pH 8.0, about pH 8.2, about pH 8.4, about pH 8.6, about pH 8.8, or about pH 9.0.

The metal nanoparticles formed according to the disclosed synthetic method are formed *in situ.*

The metal nanoparticles formed according to the disclosed synthetic method are formed - after gelation (step (c)) - *in situ* through irradiation with light, preferably ultraviolet (UV) light, for minutes to hours, preferably 1 to 60 minutes, even more preferred 1 to 10 minutes. The irradiation with light may be for from about 1 to about 60 minutes, from about 1 to about 50 minutes, from about 1 to about 40 minutes, from about 1 to about 30 minutes, from about 1 to about 20 minutes, from about 1 to about 15 minutes, from about 1 to about 10 minutes, or from about 1 to about 5 minutes. The irradiation with light may be for about 1 minute, about 10 minutes, about 20 minutes, about 30 minutes, about 40 minutes, about 50 minutes, or about 60 minutes.

The *in situ* reduction of the disclosed method may be achieved through a reducing agent. The reducing agent may be a biocompatible reducing agent, such as citric acid or sodium ascorbate. The reducing agent may be added before or after gelation (i.e. before or during step (c) or after (c)), wherein, when a reducing agent is added, the irradiation step (d) can be omitted. The method of the invention includes the irradiation step (d).

As discussed above, the present disclosure provides a nanoparticle-containing hydrogel.

Said nanoparticle-containing hydrogel is prepared by the disclosed *in situ* method.

As discussed above, the present disclosure provides the use of the disclosed nanoparticle-containing hydrogel.

The disclosed nanoparticle-containing hydrogel can be used as a device for metal delivery, preferably for sustained or controlled release metal delivery.

The disclosed nanoparticle-containing hydrogel can be used as an antimicrobial agent.

The disclosed nanoparticle-containing hydrogel can be used in a method of treatment of an infectious disease, wherein, preferably, said infectious disease is caused by Gram-positive bacteria, Gram-negative bacteria, yeast or fungi.

The disclosed nanoparticle-containing hydrogel can be used as a wound dressing and for wound healing. The nanoparticle-containing hydrogel offers great potential for application in wound healing, in particular after surgery, in treatment of chronic and large surface wounds, like diabetic ulcer or severe burns, due to its potential to reduce inflammation and its ease of application.

The disclosed nanoparticle-containing hydrogel can be used in a surgical application.

The disclosed nanoparticle-containing hydrogel can be used to prevent bacterial attachment and/or formation of microbial biofilm.

The disclosed nanoparticle-containing hydrogel can be used for topical medical application.

The disclosed nanoparticle-containing hydrogel can be used for bioimaging (by utilizing the fluorescence of the metal nanoparticles).

The disclosed nanoparticle-containing hydrogel can be used in combination with medical devices, such as stents, prostheses, catheters, dental implants, orthopedical devices, orthopedical implants.

The disclosed nanoparticle-containing hydrogel can be used for application where the hydrogel is used as a (dried) membrane, such as filtration.

Advantageously, the disclosed hydrogels may not require additional chemical modification to display antibacterial properties.

As discussed above, the present disclosure provides a device for metal delivery.

Said device for metal delivery comprises a disclosed nanoparticle-containing hydrogel. Said device for metal delivery is preferably for sustained or controlled release metal delivery.

Advantageously, the disclosed hydrogels may display sustained release of the metal nanoparticles over a long period, for example, more than 10 days.

Further advantageously, the disclosed hydrogels may be able to fully release the metal nanoparticles, even while using a low concentration of metal salt precursor.

Also advantageously, the disclosed hydrogels may only require low concentrations of metal salt precursor.

As discussed above, the present disclosure provides an antimicrobial agent comprising a disclosed nanoparticle-containing hydrogel.

As discussed above, the present disclosure provides a wound dressing or wound healing agent comprising a disclosed nanoparticle-containing hydrogel.

Said wound dressing or wound healing agent is preferably used to prevent microbial infection and/or formation of microbial biofilm.

Advantageously, the disclosed hydrogels may be able to prevent bacterial attachment, and therefore prevent the formation of biofilm.

Advantageously, the disclosed hydrogels may be able to entrap up to 99.9% water. Therefore advantageously, the moisture from the disclosed hydrogels may prevent unwanted adhesion when applied to wound sites, facilitating non-traumatic removal of the dressing.

The disclosed hydrogels offer great potential for application in wound healing, in particular after surgery, in treatment of chronic and large surface wounds, like diabetic ulcer or severe burns, due to its potential to reduce inflammation and its ease of application.

As discussed above, the present disclosure provides a pharmaceutical or cosmetic composition comprising a disclosed nanoparticle-containing hydrogel.

The disclosed pharmaceutical or cosmetic composition may be provided in the form of a topical gel or cream, a spray, a powder, or a sheet, patch or membrane. The disclosed pharmaceutical or cosmetic composition may be provided in the form of an injectable solution. The disclosed pharmaceutical or cosmetic may further comprise a pharmaceutically active compound and/or a pharmaceutically acceptable carrier.

As discussed above, the present disclosure provides the disclosed nanoparticle-containing hydrogel for use in a method of treatment of an infectious disease.

Said method comprises the step of administering an effective amount of a disclosed nanoparticle-containing hydrogel or a disclosed pharmaceutical composition to a person or subject in need thereof. Preferably, said infectious disease is caused by Gram-positive bacteria, Gram-negative bacteria, yeast or fungi.

As discussed above, the present disclosure provides the disclosed nanoparticle-containing hydrogel for use in a method of treatment of a wound and for wound healing.

Said method comprises the step of applying an effective amount of a disclosed nanoparticle-containing hydrogel or a disclosed pharmaceutical composition to a wound.

Advantageously, the disclosed hydrogels may be flexible and therefore may be able to be applied across awkward or non-flat positions on the skin.

The nanoparticle-containing hydrogel offers great potential for application in wound healing, in particular after surgery, in treatment of chronic and large surface wounds, like diabetic ulcer or severe burns, due to its potential to reduce inflammation and its ease of application.

As discussed above, the present disclosure provides a bioimaging device comprising a disclosed nanoparticle-containing hydrogel for *in vitro* and/or *in vivo* use.

Said bioimaging device is preferably for oral application, for injection and/or for topical application.

The nanoparticle-containing hydrogel offers great potential for application in wound healing, in particular after surgery, in treatment of chronic and large surface wounds, like diabetic ulcer or severe burns, due to its potential to reduce inflammation and its ease of application.

As discussed above, the present disclosure provides a kit, comprising
(i) at least one peptide as defined herein, optionally, in an aqueous solution;
(ii) a precursor of metal nanoparticles as disclosed above, preferably a metal salt solution (such as AgNO₃);
(iii) instructions for gel formation to obtain a peptide hydrogel and for irradiating the peptide hydrogel resulting therefrom for obtaining a nanoparticle-containing hydrogel;
(iv) optionally, excipients.

As discussed above, the present disclosure provides a kit, comprising
(i) a hydrogel obtained from at least one peptide as defined herein, preferably in a container or containment to be protected from (any kind of) radiation;
(ii) a precursor of metal nanoparticles as disclosed above, preferably a metal salt solution (such as AgNO₃);
(iii) instructions for irradiating the peptide hydrogel for obtaining a nanoparticle-containing hydrogel;
(iv) optionally, excipients.

In the disclosed nanoparticle-containing hydrogels, the interactions between the functional groups of the peptide chains and metal nanoparticles (such as silver nanoparticles) may complement each other, resulting in a controlled *in situ* metal nanoparticle synthesis. Additionally, the fibrous structure of the ultrashort peptide hydrogels aids in minimizing the aggregation of metal nanoparticles synthesized *in situ* through the reduction of metal ions via UV irradiation while the metal nanoparticles help to stabilize the structure of the hydrogels. Advantageously, the disclosed nanoparticle-containing hydrogels may not require additional chemical modification.

The disclosed method for *in situ* synthesis of nanoparticle-containing hydrogel may be easy to follow and fast as it may only take about 1 minute of UV irradiation in one embodiment for the reduction of metal ions into metal nanoparticles. In contrast to other proposed biomaterials which require up to 12, 3 and 1 hour respectively to reduce the metal ions to metal nanoparticles in their biomaterials, the disclosed hydrogels may be prepared more efficiently, saving production time. The disclosed protocol for *in situ* synthesis of nanoparticle-containing hydrogel also allows a reproducible synthesis of uniformly distributed metal nanoparticles within the fibrous structure of the hydrogels, allowing the disclosed hydrogels to exert their anti-microbial activities over the entire applied area effectively.

Using the disclosed synthetic protocol, metal ions may be reduced to metal nanoparticles when the need to use the disclosed hydrogel arises. This ensures consistency in the quality and quantity of metal nanoparticles in the disclosed hydrogels.

### Fluorescent metal nanoparticles or nanoclusters for bioimaging

Fluorescent metal nanoparticles such as gold (Au), silver (Ag) or copper (Cu) nanoparticles may be used in bioimaging as replacements for quantum dots and organic dyes. Quantum dots are generally highly toxic due to their cadmium content, whereas organic dye molecules often suffer from photo-bleaching. Fluorescent metal nanoparticles may therefore present a good alternative to these bioimaging agents.

By combining the anti-microbial activity of silver nanoparticles together with their possible fluorescence (dependent upon nanoparticle sizes and surface molecules) the disclosed hydrogels may be useful in bioimaging and mechanosensors.

### Further description of preferred embodiments

We have previously introduced a novel class of ultrashort peptides, containing 3-7 amino acids, with an innate self-assembly capacity for self-assembly. The peptide monomers form helical fibers that entangle to three-dimensional networks and eventually entrap water to form hydrogels (Hauser *et al.,* 2011; Mishra *et al.,* 2011). These hydrogels show remarkable properties with regards to mechanical stiffness, elasticity and biocompatibility and have been successfully employed in diverse applications as biomimetic material (Lakshmanan *et al.,* 2013). However, earlier attempts of physically entrapping a bioactive compound within the self-assembling peptide hydrogel resulted in a burst release - a sustained release was never achieved. Hence the goal of this study was to develop an organic-inorganic hybrid system that preferably enabled an initial burst release, followed by a sustained release of the bioactive nanoparticle for wound healing applications.

In particular, in this disclosure, we report the development of a novel silver-impregnated biomaterial made of ultrashort peptides which only contain aliphatic amino acids. This unique class of ultrashort peptides is able to self-assemble in water and in physiologically relevant buffers to form hydrogels (Hauser *et al.,* 2011; Mishra *et al.,* 2011). The gel made from peptide Ac-LIVAGK-NH₂ (Ac-LK₆-NH₂) was used as a matrix for *in situ* Ag NP synthesis with silver nitrate as the Ag(I) source. Under UV light, the silver was reduced in a very short amount of time, without the presence of any other reducing agent. The morphological and mechanical properties of the resulting hybrid biomaterial were examined by transmission electron microscopy (TEM) and rheology and its antibacterial properties were evaluated. In addition, biocompatibility studies were performed using human primary dermal fibroblasts for evaluating suitability of the hybrid biomaterial for wound healing applications.

### Discussion

The inventors have explored the possibility of adding antibacterial properties to ultrashort peptide hydrogels by incorporating *in situ* formed silver nanoparticles. The porous character of the fibrous peptide hydrogel and the non-reactivity of the functional groups of the peptide chains support a controlled *in situ* formation of the silver nanoparticles. Furthermore, the easy way of silver nanoparticle formation gives advantages over previous reported systems. Unlike other reported antibacterial hydrogels, such as porous chitosan blended with PEG (Vimala *et al,* 2010) and electrospun gelatin fiber mats (Rujitanaroj *et al.,* 2008), Ag-Ac-LK₆-NH₂ hydrogels do not require additional chemical modification.

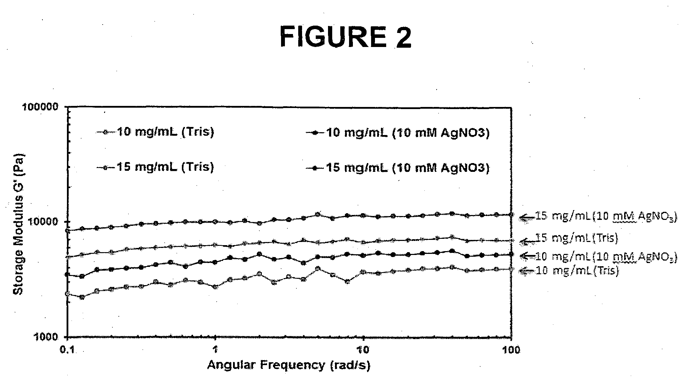

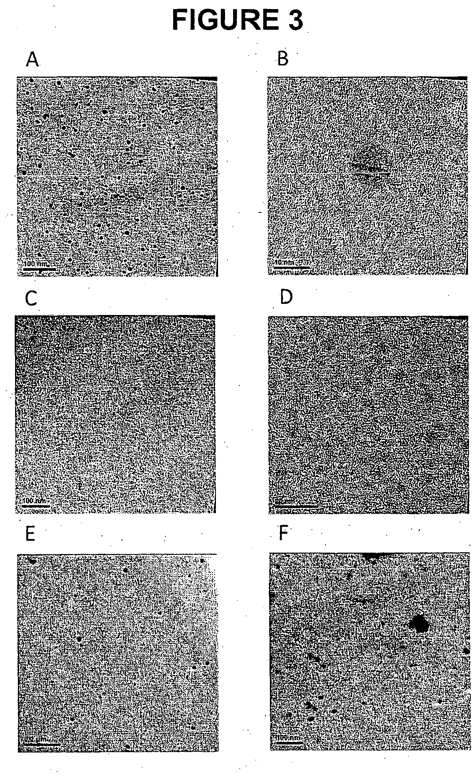

The successful synthesis of monodispersed Ag NP was confirmed by TEM microscopy showing an average size distribution of 10-20 nm (Figure 3A to D) and a homogenous distribution throughout the measured samples. The same holds true for sample measured before (Figure 3E) and after expose to bacterials (Figure 3F). The synthesis of small, monodispersed nanoparticles is crucial for a material with antibacterial properties. Recent studies by Martinez-Gutierrez *et al.* (2010) have shown that the ideal size for antibacterial Ag NP is below 25 nm. We are well within this range. Interestingly, we observed, that the hydrogel and the Ag NPs show a cooperative effect in terms of the mechanical properties of the hydrogel compared to a gel without Ag NP (Figure 2). Rheology studies showed that gels containing 10 mM of Ag NP have a storage modulus almost twice as large as samples of the same concentration but without Ag NP. Similar to observations with biomineralization of hydrogels with the presence of salt crystals, the presence of silver nanoparticles within the gel lead to an substantial increase of mechanical stiffness. A sustained release was observed for up to 14 days, whereby a faster release is present in the first 48 h. The release of Ag NP is crucial for the ability of the new material in order to act as an antibacterial agent. Only when Ag NP is released into the surrounding of the hydrogel, an effective bacterial treatment will be observed. Furthermore, the faster release of Ag NP in the beginning delivers a higher local concentration of the antibacterial agent, helping to quickly reduce inflammation caused by bacterial infection. Additionally, a sustained release over the following 12 days helps to prevent reinfection.

While sustained release of silver nanoparticles over 2 weeks has been reported, the water-soluble polyethylene glycol (PEG) polymers containing reactive catechol moieties were able to release approximately only 1% of the total amount of silver inside the hydrogel disks (Fullenkamp et al., 2012). In contrast, our Ag-Ac-LK₆-NH₂ hydro gels (100 µL) are able to release up to 90 % of the *in situ* synthesised silver nanoparticles while using a much lower concentration as previous reported systems.

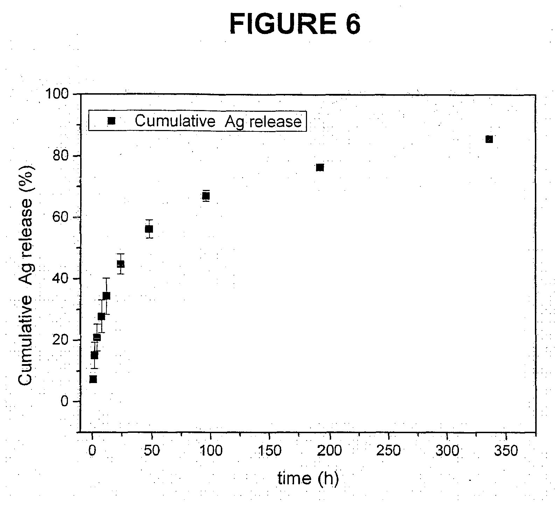

The ability of the new material to inhibit bacteria growth was tested using two different methods, the disc diffusion method and the suspension method. Both methods showed a similar trend with regards to growth inhibition, and thus confirmed each other's result. It is very interesting to note, that the Ag NP were most effective against *P. aeruginosa,* a bacteria strain known for its drug resistance, which can be seen by the reduced activity of tetracycline control in *P. aeruginosa* compared to S. *aureus* and *E. coli.* For both methods a killing of *P. aeruginosa* was observed, indicated by a clear circular surrounding zone when using the disc diffusion method. Also, a reduction in the starting optical density (OD) was seen using the suspension method (Figure 6). Taking into account, that *P. aeruginosa* is very hard to treat and thus can lead to severe health problems upon infection, the development of efficient antimicrobial compounds is a highly important issue. Based on the obtained bacterial inhibition data, Ag-Ac-LK₆-NH₂ could offer new valuable new opportunities in the treatment of wound infections. This would overcome the need of oral or intravenous application of traditional antibiotics and could help to reduce health cost.

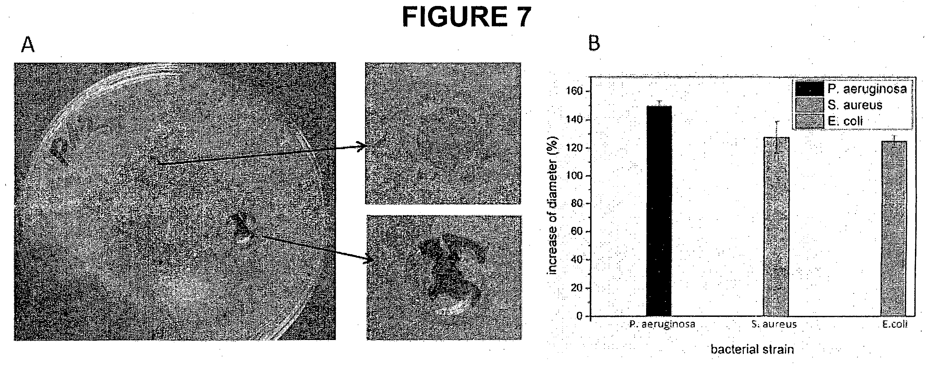

To characterize, if the newly developed biomaterial showed solely bacterial killing efficacy but no mammalian cell toxicity, the biocompatibility was tested *in vitro.* For this purpose, primary HDFa cells were used and incubated with the Ag-Ac-LK₆-NH₂ in a transwell system. This experimental set-up demonstrated that the cells are exposed to the Ag NP. We propose our Ag NP hydrogel as an efficient wound dressing device, where the Ag NPs are released to the wound. The result of the biocompatibility study is shown in Figure 7, showing cell viability after 24 and 48 h. At both time points, a reduction in the cell viability was observed by about 20 %. However, live/dead assays did not show a significant number of dead cells when compared with the untreated control (Figure 7b-d). Using 1% agar gels as a control, we observed that the agar control almost matched the values obtained by the 1% peptide hydrogels. When an empty transwell was placed used, a reduced viability was observed after 24 and 48 h. However, even the empty transwell showed about 10 % reduction in cell viability after 48 h of incubation (Figure 7a). These results let us to the conclusion, that the transwell set-up changes the cell environment and thus influences the cell proliferation rate, but not the viability which can be seen from the live dead assay analysis. Most likely, nutritional factors from the media are sustained in the gel, agar or transwell membrane matrix, leading to a reduced cell proliferation. Given these results, Ag-Ac-LK₆-NH₂ hydrogels appear to be able to efficiently kill bacteria without significantly altering the cell viability.

### Conclusions

Anti-bacterial hydrogels containing *in situ* formed silver nanoparticles were developed using ultrashort self-assembling peptides and silver nitrate. The conversion of silver nitrate to silver nanoparticles needed only the presence of UV light. The *in situ* Ag NP formation was accomplished after hydrogel formation, enabling with the porous hydrogel structure a controlled particle size after reduction of Ag(I) to Ag NP. This method allows the formation of monodispersed Ag NP without the addition of an external reducing agent. The Ag NP formation was characterized by TEM, demonstrating that monodispersed particles with less than 20 nm could be formed. The nanoparticles were also uniformly distributed within the measured sample. The Ag NP containing hydrogel showed increased mechanical strength when compared to the peptide hydrogel alone. We conclude that the presence of silver nanoparticles within the soft matter hydrogel results in an increase in the mechanical stiffness of the hydrogel. Anti-bacterial tests were conducted using three bacterial strains namely, *E.coli, P. aeruginosa* and *S. aureus.* Highly efficient bacterial growth inhibition was observed for all three bacteria strains, using as little as 10 mM silver nanoparticle hydrogels. This is significantly less amount than what was reported with other systems. It is important to note, that the best results were obtained for *P*. *aeruginosa* which is known for its multidrug resistance and thus very difficult to treat in patients. Biocompatibility studies on HDFa cells showed that the novel biomaterial does not significantly influence the cell viability. The silver concentration is obviously within the therapeutic window, but low enough to not significantly affect mammalian cell viability. This novel silver-releasing biomaterial offers great potential for application in wound healing, in particular after surgery, in treatment of chronic and large surface wounds, like diabetic ulcer or severe burns, due to its potential to reduce inflammation and its ease of application.

### Further advantages and comparisons

The silver release studies described herein demonstrated a sustained release of silver over 12 days. This property of the disclosed Ag-Ac-LK₆-NH₂ hydrogels allows them to function as bactericidal agents and to prevent bacterial biofilm formation which can hinder wound healing. Current technology in Silvazine™ requires daily change of dressing, while Acticoat™ only offers up to 7 days of bacterial protection. Besides cost reduction due to a decrease in frequency in changing wound dressing, the disclosed Ag-Ac-LK₆-NH₂ hydrogels also boosts convenience for both users and clinicians. While sustained release of silver nanoparticles over 2 weeks has previously been reported for water-soluble polyethylene glycol (PEG) polymers containing reactive catechol moieties, these polymers were only able to release approximately 1% of the total amount of silver inside the hydrogel disks. In contrast, and as discussed herein, the disclosed Ag-Ac-LK₆-NH₂ hydrogels (100 µL) are able to fully release the *in situ* synthesized silver nanoparticles.

The disclosed hydrogels may be able to entrap up to 99.9% of water. On the other hand, Acticoat™ 7 and Acticoat™ Flex require continual external moistening over the entire course of healing. As it is crucial to provide a moist environment to optimise the wound healing process, the disclosed Ag-Ac-LK₆-NH₂ hydrogels may be clinically superior and more user-friendly.

All commercially available wound management products under Silvazine™ and Acticoat™ are adhesive to the newly formed skin to varying extent except for Acticoat™ Absorbent which makes use of the exudate from the wound to form a gel, thereby minimising adhesion. However, not all wounds produce sufficient exudate for the formation of gels and in cases where there is insufficient moisture, substantial attachment to skin may still occur. Comparatively, the moisture from the Ag-Ac-LK₆-NH₂ hydrogels prevents unwanted adhesion, facilitating non-traumatic removal from the wound and is clinically preferred.

The disclosed Ag-Ac-LK₆-NH₂ hydrogels are also easier to use because they can be applied across awkward positions. While improvements have been made to design wound therapy coats that are less rigid such as Acticoat™ Flex, it is understood that solid meshes are not as flexible as the disclosed Ag-Ac-LK₆-NH₂ hydrogels. Being flexible, the disclosed Ag-Ac-LK₆-NH₂ hydrogels can be applied much more conveniently, saving productive time.

### Brief Description Of Drawings

The accompanying drawings illustrate a disclosed embodiment and serves to explain the principles of the disclosed embodiment. It is to be understood, however, that the drawings are designed for purposes of illustration only, and not as a definition of the limits of the invention.
**Figure 1** shows hydrogels synthesised with AgNO₃ solutions. (A) Ag+ ions present in AgNO₃ solution were reduced to Ag(0) with UV radiation, resulting in colour change from colourless to brown in the hydrogels (from left to right: 0 mM AgNO₃; 10 mM AgNO₃; 20 mM AgNO₃). (B) Peptide was pipetted into gel cast, allowed to gel and then irradiated with UV.
**Figure 2** is a graph showing the rheological characterization of ultrashort peptide hydrogels with and without silver nanoparticles at 10 and 15 mg/mL. Frequency sweep was performed at 0.1% strain.
**Figure 3** is a series of transmission electron microscope (TEM) images of the silver nanoparticles distribution in Ag-Ac-LK₆-NH₂ hydrogels formed in water using 10 mM (A, B) and 20 mM (C, D) AgNO₃ solution and a 10 mM solution in Tris buffer at pH 8.4 before (E) and after (F) incubation with bacteria. (A, C) Uniform distribution of silver nanoparticles. (B, D) Size of silver nanoparticle is approximately 10 - 20 nm.
**Figure 4** is a graph showing the cumulative release of silver from 100 µL of Ag-Ac-LK₆-NH₂ hydrogels as a function of time.
**Figure 5** depicts an assessment of anti-microbial properties of Ag-Ac-LK₆-NH₂ hydrogels
   (A) Preliminary testing of Ag-Ac-LK₆-NH₂ hydrogels formed with different [AgNO₃] solutions and their potencies against the bacteria;
   (B) Disk diffusion method;
   (C) Bacterial suspension with visible bacterial attachment on controls (second row) and no bacterial adhesion onto Ag-Ac-LK₆-NH₂ hydrogels (first row). *Bacilus subtilus* also attached more strongly to the controls (compare column 2 and 3).
   (D, E) Average optical density of the bacterial suspension after 24 hours incubation of *Bacilus subtilus* and *E.coli* respectively.
**Figure 6** is a graph showing the cumulative release of silver from 100 mL of Ag-Ac-LK₆-NH₃ hydrogels as a function of time.
**Figure 7** depicts an assessment of anti-microbial properties of Ag-Ac-LK₆-NH₂ hydrogels by disk diffusion.
   (A) Comparison of Ag-Ac-LK₆-NH₂ hydrogels and Ac-LK₆-NH₂ hydrogels on an agar plate covered with *Pseudomonas aeruginosa;*
   (B) Graphical presentation of the resulting radius measurement in comparison with Ac-LK₆-NH₃ hydrogels alone.

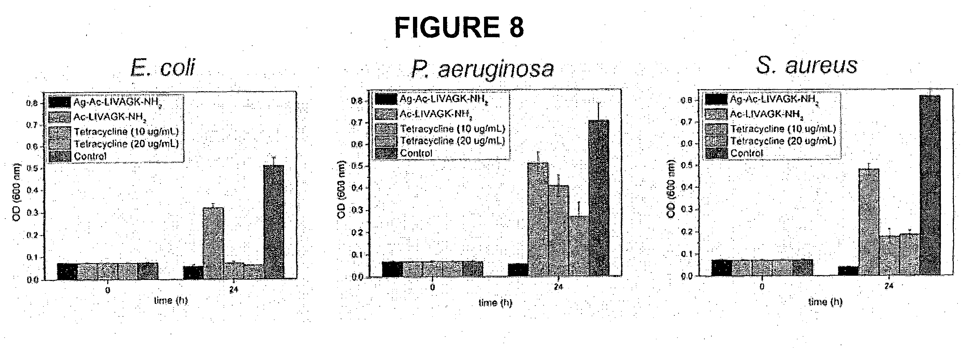

**Figure 8** shows a series of graphical presentations of the OD measurements in *E. coli, P. aeruginosa* and *S. aureus* at t = 0 and t = 24. The bars are arranged according to the order in the figure legends, i.e. (from left to right) Ag-Ac-LIVAGK-NH₂, Ac-LIVAGK-NH₂, Tetracycline (10 µg/mL), Tetracycline (20 µg/mL), and Control.

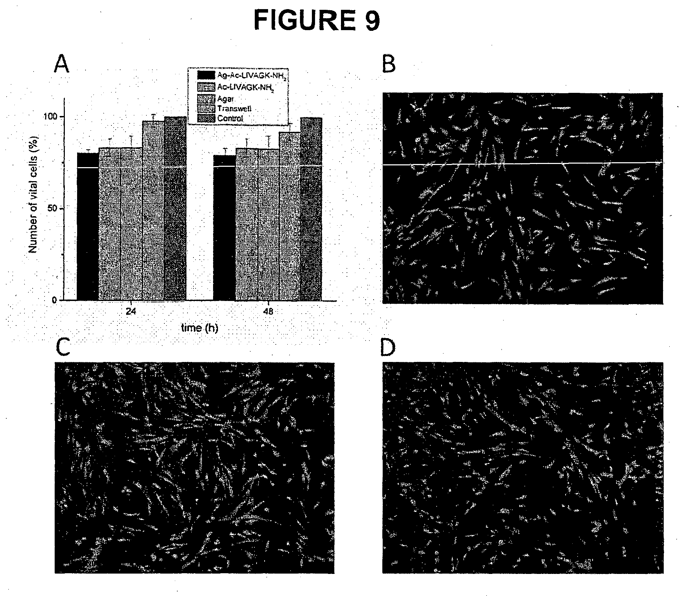

**Figure 9** shows a graphical presentation of the biocompatibility normalized to untreated cells (A), live dead image of cells treated with Ag-Ac-LK₆-NH₂ (B), Ac-LK₆-NH₂ (C), or untreated cells (D) respectively. The bars are arranged according to the order in the figure legends, i.e. (from left to right) Ag-Ac-LIVAGK-NH₂, Ac-LIVAGK-NH₂, Agar, Transwell, and Control.

### Examples

Non-limiting examples of the invention and a comparative example will be further described in greater detail by reference to specific Examples, which should not be construed as in any way limiting the scope of the invention.

### 1. Materials and Methods

### 1.1 Materials for Peptide Synthesis

Fmoc-lys-rink resin (resin 0.42 mg/mol), Fmoc protected amino acid i.e. glycine, alanine, valine, leucine and isoleucine and O-(benzotriazol-1-yl)-N,N,N',N'-tetramethyluronium tetrafluoroborate (TBTU) were purchased from GL Biochem (Shanghai) Ltd. Dimethylformamide (DMF) (analytical grade) was purchased from Fisher Scientific. Acetic anhydride (Ac₂O) and dimethyl sulfoxide (DMSO) was purchased from Sigma Aldrich. *N*,*N*-Diisopropylethylamine (DIPEA), dichloromethane (DCM), trifluoroacetic acid (TFA) and TIS (triisopropylsilane) were purchased from Alfa Aesar. Piperidine and silver nitrate (AgNO₃) were purchased from Merck Schuchardt OHG. Diethyl ether (Et₂O) was purchased from Tedia Company Inc.

Ac-LIVAGK-NH₂ (Ac-LK₆-NH₂) was purified on an Agilent 1260 Infinity preparative HPLC system equipped with a Zorbax SB-C18 column (21.2 mm × 150.0 mm Ø 7 µm). The HPLC was coupled over an active splitter to a SQ-MS for mass triggered fraction collection. MilliQ water and HPLC grade acetonitrile, both containing 0.1% formic acid, were used as eluents, and a solvent gradient was used to elute the pure peptide. ESI-MS spectra were measured in positive mode on an Agilent 6130 Quadrupole LC/MS system equipped with an ESI spray chamber and coupled to a preparative Agilent 1260 HPLC unit.

### 1.2 TEM Analysis

TEM analysis was performed with a Philips CM300 FEG TEM at 300 kV. TEM images were analyzed using Gatan Digital Micrograph. Samples were freeze dried prior to analysis, and then resuspended in EtOH and dropped on a carbon-film Cu grid for TEM imaging.

### 1.3 Peptide Synthesis and Purification

Ac-LK₆-NH₂ peptide was synthesised using solid phase peptide synthesis. Briefly, Fmoc-lys-rink resin was weighed out and the beads were allowed to swell for an hour in DMF. Subsequently, 10 equivalents of Ac₂O and DIPEA were added to prevent reaction of the side-groups, and the mixture was allowed to incubate for 45 minutes. The resin was then washed with DMF before going through a series of deprotection reactions using 20% piperidine in DMF and coupling reactions with the addition of 4 equivalents of the desired amino acid along with 4 equivalents of TBTU and 4 equivalents of DIPEA. Acetylation of the N-terminus was performed using a 4 times excess of Ac₂O and DIPEA. Following this, the resin was washed with DCM and allowed to dry, before cleaving the peptide from it using a mixture of 95% TFA, 2.5% water and 2.5% TIS. The solvents were removed under reduced pressure and Et₂O was added to precipitate the peptide. The peptide was isolated by centrifugation, washed twice with Et₂O and dried under reduced pressure. Purification was performed in a preparative high-performance liquid chromatography system coupled to an electron spray ionization mass spectroscopy equipment (HPLC-ESI-MS) (Agilent Technologies, 1260 Infinity Series). The peptides were introduced into the HPLC-MS system by completely dissolving them in a minimum amount of DMSO. Yield: 3.4 g (60 %), ESI-MS: Calculated for C₃₀H₅₇N₈O₇ ([M+H⁺]⁺) 641.43, Found: m/z 641.5.

### 1.4 Hydrogel preparation

All solutions for gel preparation used Tris buffer (100 mM, pH 8.5) that was prepared from Tris base and brought to a pH of 8.5 with 1 M nitric acid. The standard procedure for preparation of the hydrogel involved the addition of requisite amount of AgNO₃ solution prepared using the Tris buffer to weighed out samples of Ac-LK₆-NH₂ to achieve the desired concentration. Briefly, to prepare 10 mg/mL of Ac-LK₆-NH₂ hydrogel, 1 mL of AgNO₃ solution was added to 10 mg of peptide. After gelation in the dark, the hydrogel was subjected to UV radiation at 254 nm for approximately 5 minutes using a UV-lamp.

### 1.5. Oscillatory Rheometry

Viscoelasticity of the peptide hydrogels was measured using an ARESG2 rheometer (TA Instruments). A serrated stainless steel, parallel-plate geometry of 8-mm diameter was used and the gap distance was maintained between 0.8 and 2 mm. Oscillatory frequency sweep studies were performed for a range of 0.1-100 rad/s, using a 0.1% strain. Oscillatory amplitude sweep studies were conducted from 0.01 to 100% strain with an angular frequency of 1 rad/s. All measurements were done at 25°C, at 1d post gelation.

### 1.6 Determination of silver release by inductively coupled plasma mass spectrometry

Silver release studies were carried out in 24-well transwell plates (Corning Transwell® 6.5 mm, 8.0 µM with a polycarbonate membrane insert) using 100 µL of gel in the transwell and 400 µL of PBS solution in the peripheral well. For this purpose, Ag NP containing gels were prepared in Tris buffer (100 mM, pH = 8.5, containing 10 mM of AgNO₃) and 100 µL of the resultant solution was added into the transwell. The gel was allowed to cure overnight, after which, the Ag NPs were formed by exposing the gels to UV light (254 nm for 5 mins). Then, the transwells were placed into the 24 well plate and 400 µL of PBS solution was added into the peripheral well. Samples were taken at different time points and digested with nitric acid (68% suprapur, Merck) in a 1:1 ratio by volume overnight. After dilution of the samples, the drug release was determined by ICP-MS analysis. At the end of the release study, the residual gels were dissolved in 400 µL PBS to determine the total drug concentration.

All ICP-MS analyses were performed on a Perkin Elmer Elan DRC II instrument and the ¹⁰⁷Ag isotope was used for quantification. Calibration was conducted using an external standard (Sigma-Aldrich) between 1-200 ppb. The concentrations of the samples were calculated based on the calibration curve plotted using Elan software, and was used to calculate the accumulative release profile as a percentage.

### 1.7 Bacterial growth inhibition assay

### Preparation of Ac-LK₆-NH₂/Ag NP hydrogels for bacterial suspension assays

Gels were prepared in 24-well sterile Nunc plates. 10 mM AgNO₃ solution in 100 mM Tris-buffer (pH = 8.5) was freshly prepared and used to dissolve the peptide to a final concentration of 10 mg/mL. The resultant solution was sonicated for 15-20 s to ensure complete dissolution of the peptide. 400 µL of the peptide solution was then aliquoted into each well and allowed to spread evenly on the surface. The Ac-LK₆-NH₂ concentration was chosen to allow a clear, solid hydrogel to form in a reasonable time-frame while allowing uniform aliquoting of the peptide solution into the wells of the plate. Although gelation of Ac-LK₆-NH₂ occurred within minutes in Tris-buffer at 10 mg/mL, the plates were left to stand overnight in the dark to ensure complete gelation. Prior to use, the plates were exposed to short-wavelength UV at 254 nm for 5 minutes to allow Ag NP formation. For the control gels, 100 mM Tris buffer without AgNO₃ was used for Ac-LK₆-NH₂ hydrogel preparation.

The concentration of silver was selected so that the desired killing efficiency could be achieved using as little material as possible. Ac-LK₆-NH₂ hydrogels with different concentrations of silver i.e. 1 mM, 10 mM, 25 mM and 50 mM were evaluated for their efficacy in killing bacteria via the disc diffusion method. It was found that compared to the 10 mM gels, the 25 mM and 50 mM gels had marginally higher efficiencies while using much higher amounts of silver. Therefore, the 10 mM concentration was considered optimal and used for subsequent *in vitro* evaluations.

### Bacterial Suspension Assays

50 µL of bacterial stock was inoculated overnight in 5 mL of Luria-Bertani (LB) medium (pH = 7.5), with constant shaking at 300 rpm at 37°C. The log-phase bacterial solution was then diluted in LB medium to obtain an O.D. of 0.07 (at 600 nm). This was used as the starting bacterial solution (0 hr reading) and 400 µL of the solution was dispensed into each well of the 24-well plate containing the hydrogels. Tetracycline antibiotic (Sigma Aldrich, USA), at a final concentration of 10 and 20 uM was used as the positive control. Ac-LK₆-NH₂ hydrogels without Ag NPs and bacteria-only solutions served as the negative controls. The plates were wrapped in aluminium foil and placed at 37°C under 50 rpm rotation in a shaker-incubator for 24 hrs. For the O.D. readings, 100 µL of the bacterial solution was transferred into a transparent 96 well Nunc plate just before measurement, to prevent the hydrogel layer from influencing absorbance measurements. The assay was performed in triplicates, with three replicates for each independent experiment.

### Ring-cast method for preparing hydrogels

For rheometry and disc-diffusion experiments, a ring-cast method was used for hydrogel preparation to ensure uniform gel volume and facilitate easy transfer of the intact gel. After dissolving the peptide, 200-µL aliquots of the solution were immediately transferred into plastic ring casts of 9-mm diameter placed on clean 35-mm tissue-culture dishes. The top and bottom of each ring cast was covered with a layer of parafilm, and the tissue-culture dish was tightly sealed with parafilm to prevent evaporation. The gels were allowed to cure overnight and NP formation was achieved using UV light for about 5 minutes.

### Disc-Diffusion Method for evaluating anti-bacterial properties

150-µL of log-phase bacterial stock was uniformly spread on LB-Agar (1.5% agar) plates and incubated until a bacterial biofilm was formed. The hydrogel discs were placed on top of the film and zones of inhibition/killing were measured at 24 hours. The diameter of the zone of inhibition was measured using a pair of vernier calipers. All experiments were performed in triplicates.

### 1.8 Cell Culture

Human Dermal Fibroblasts - Adult (HDFa) were purchased from Lonza. The cells were cultured in MEM with 2mM L-glutamine and Earle's salts (500 mL) supplemented with 10% heat inactivated fetal bovine serum, 1% of a mixture of penicillin and streptomycin (Invitrogen, CA, U.S.A), Sodium bicarbonate (1.5 g/L final concentration), NEAA (5mL of 100X stock) and Sodium pyruvate (5 mL of 100 mM stock). The cells were maintained either in a T175 or T75 cell culture flask (Nunc) at 37 °C in a humidified incubator with 95% air and 5% CO₂.

### Biocompatibility Study

Biocompatibility studies were carried out in 24-well transwell plates (Corning Transwell® 6.5 mm, 8.0 µM with a polycarbonate membrane insert) using 100 µL of gel in the transwell and 400 µL of medium in the peripheral well. Cell viability was measured after 24 h and 48 h of incubation.

HDFa cells (12,000 cells/well) were seeded into a 24-well plate and incubated for 24 h in 400 µL medium. In the interim, 100 µL hydrogel-containing transwells were prepared. For this purpose, Ac-LK₆-NH₂ was weighed out, and tris buffer (100 mM, pH = 7.5) containing 10 mM AgNO₃ was added to obtain a total concentration of 10 mg/mL. 100 µL of the prepared solution was added into the transwell and allowed to gel for 4 h at 37 °C. As a control Ac-LK₆-NH₂ hydrogels were prepared the same way, but without AgNO₃. For the positive controls, cells without any hydrogel, cells with transwells containing 100 µL of 1% agar gels and cells with empty transwells were used. Prior to inserting the hydrogel containing transwells into the cell culture plates, Ag NPs were formed using UV light. In order to keep the samples sterile, the UV light of the laminar flow cabinet was used, and exposure time was increased to 10 minutes. Subsequently, the transwells were placed on top of the cells growing on the plates and incubated for 24 hours. Cell viability was determined by means of a colorimetric microculture assay (WST-1, Roche). The transwells were taken out of the plate, the medium was carefully removed and fresh medium containing 10% WST-1 reagent was added. After 2 h of incubation at 37 °C, 90 µL of the supernatant solution was transferred onto a 96-well plate and absorption at 450 nm was measured on a plate reader (Infinite M200, Tecan). To determine the toxicity after 48 h of incubation with the hydrogels, the WST-1 containing solution was removed from the cells, and fresh medium was added. After 1 h incubation, the medium was replaced again and the transwells were added back. After another 24 h incubation (total 48 h incubation) the cell viability was determined as described above. With the exception of the 1% agar control and the empty transwell, all experiments were carried out in independent triplicates. Controls containing 1% agar and the empty transwell were carried out in independent duplicates with at least four samples per duplicate. The biocompatibility is expressed as a percentage of survival relative to the untreated control.

### Live/Dead assay

HDFa cells were seeded in 24-well plates (12,000 cells/well) and incubated for 24 h. Afterwards, the cells were exposed for 48 h to Ag-Ac-LK₆-NH₂ hydrogel samples prepared in the transwell. Ac-LK₆-NH₂ hydrogel and untreated cells were used as controls. Incubation was done in transwell plates as described for the biocompatibility studies. After 48 h, the transwell was removed, the medium replaced with DPBS solution containing approximately 2 µM calcein AM and 4 µM ethidium homodimer-1 (LIVE/DEAD® Viability/Cytotoxicity Kit, Life Technologies™) and incubated for 40 minutes. Just before imaging, the media solution was removed for greater clarity. Live cells were imaged in the green channel and dead cells in the red channel. The obtained pictures were superimposed using ImageJ.

### 2. Results

### 2.1 Formation of hybrid hydrogels

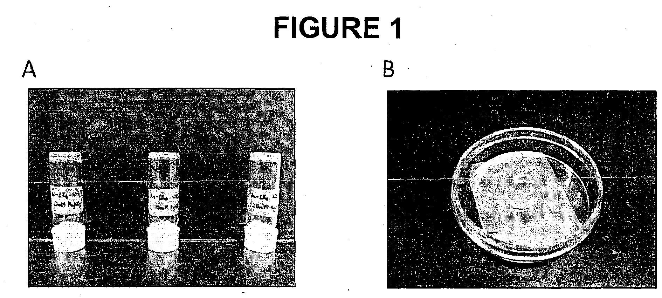

Silver nanoparticle containing hydrogels (Ag-Ac-LK₆-NH₂) were formed by the addition of AgNO₃ solution to Ac-LIVAGK-NH₂ at a basic pH of 8.5 and exposing the hydrogels to UV radiation at 254 nm for several minutes after gelation. The rate of gelation was not significantly affected by the concentration of AgNO₃ solution used. Higher peptide concentrations required shorter gelation periods i.e. 15 mg/mL Ag-Ac-LK₆-NH₂ took approximately only 1 minute for gelation as compared to 30 minutes for 10 mg/mL Ag-Ac-LK₆-NH₂, Subsequent nanoparticle formation was achieved by irradiation of the samples with UV light for several minutes. A colour change (from colourless to brown) was observed for hydrogels containing higher silver nanoparticle concentration (Figure 1A). Additionally, hydrogels containing silver nanoparticles retained the shape better (Figure 1B) than hydrogels without silver nanoparticles.

During the process of Ag NP formation, we observed that the mechanical properties of the gels changed. To quantify this observation, differences in mechanical properties between the hydrogels with and without silver nanoparticles, and also between hydrogels of different peptide concentrations were studied using oscillatory rheometry. Frequency sweep was performed from 0.1-100 rad/sec at 0.1% strain. As depicted in Figure 2, a significant difference in the storage modulus between samples with and without Ag NPs can be observed.

### 2.2 TEM images of silver nanoparticles synthesised in situ

TEM images of Ag-Ac-LK₆-NH₂ hydrogels formed using 10 mM and 20 mM AgNO₃ solution in Milli-Q were taken after freeze drying the samples. As seen in Figure 3A and 3C, a uniform distribution of the silver nanoparticles could be achieved by reduction of silver ions with UV light. In addition, the controlled reduction with UV light leads to a uniform size distribution of the Ag NP of the particles at around 10-20 nm which can be seen in Figure 3a and 3c as well as in the high resolution images in Figure 3B and 3D. Monodisperse NPs with a size range of 10-20 nm are ideal to achieve an efficient anti-bacterial activity (Martinez-Gutierrez et al., 2010). In addition, monodisperse Ag NP were observed even at higher concentrations, whereby the higher concentrations of Ag⁺ ions only result in a higher number of silver nanoparticles but not in larger particles.

### 2.3 Silver release properties of composite ultrashort peptide hydrogels

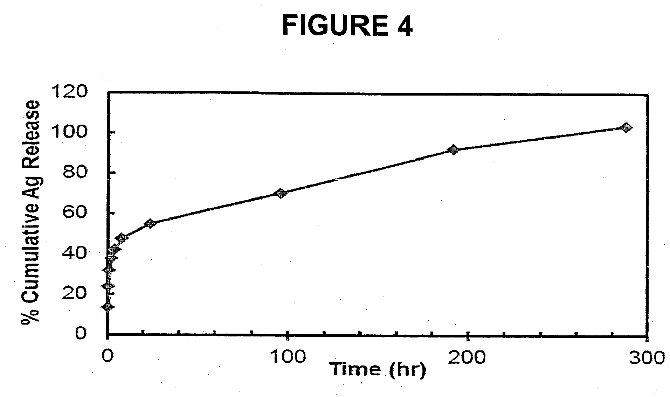

Silver release from Ag-Ac-LK₆-NH₂ hydrogels was studied in transwell plates. 100 uL of Ag-Ac-LK₆-NH₂ hydrogels in tris buffer (pH = 8.4) were immersed in PBS (pH 7.4) for up to 14 days with periodic sampling of the Ag concentration in the PBS solution. Figure 4 shows the cumulative release of silver from the hydrogels. A burst release could be observed within the first 48 h of the study which accounts for almost 50 % of the total released Ag NPs. However, a sustained release could be observed for as long as 14 days.

### 2.4. Anti-microbial activity of composite ultrashort peptide hydrogels

The anti-microbial activity of Ag-Ac-LK₆-NH₂ hydrogels was investigated in three different bacteria strains, *E.coli, P. aeruginosa* and *S. aureus* respectively by the disc diffusion method and in suspension culture. In an initial screening, Ag-Ac-LK₆-NH₂ hydrogels discs (200 µL per disc) with different concentrations of silver ions ranging from 1 to 100 mM were placed on top of an *E.coli* covert agar plate and the increase in diameter was measured after 24 h. As expected, a concentration depending increase in diameter was observed, however, we found that 10 mM of silver was sufficient to show a significant killing effect noticeable by the increase of the diameter. Keeping in mind that silver nanoparticles are potentially toxic the use of a low concentration is preferred. Based on this initial screening all further biological studies were carried out using a silver concentration of 10 mM.

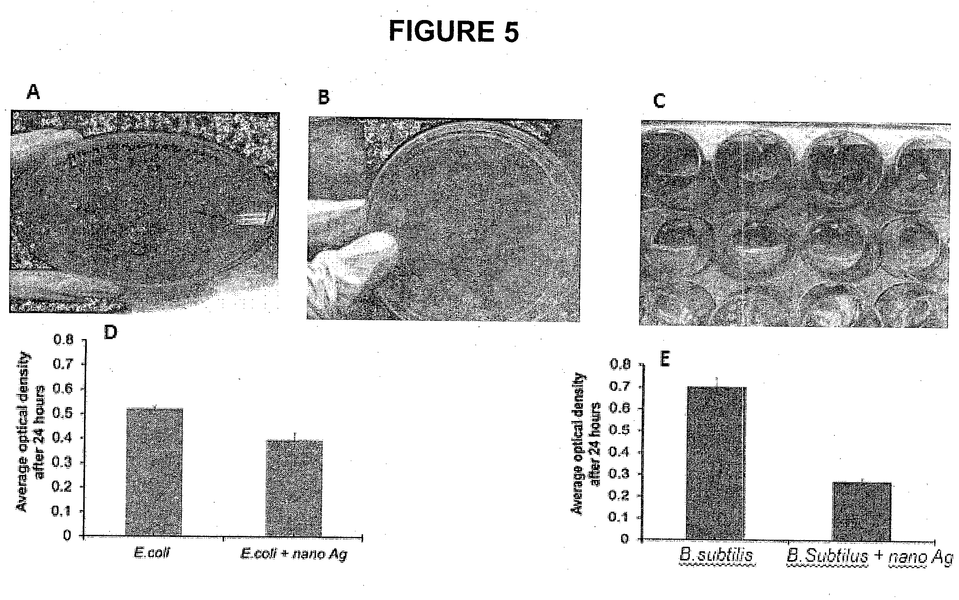

Disc diffusion tests were conducted in independent triplicates using *E.coli, P. aeruginosa* and *S. aureus* covered agar plates. The hydrogels were placed on top of the bacterial lawn and incubated at 37 °C for 24 h. After which the increase of diameter was measured with a vernier caliper and compared to Ac-LK₆-NH₂ hydrogels disc without Ag NP (Figure 5B). As shown in Figure 5A, a clear circle was formed around the Ag-Ac-LK₆-NH₂ hydrogels disc after 24 h whereas no killing was found for the hydrogels without Ag NP. When comparing the killing efficiency of Ag NP based hydrogels between *E.coli, P. aeruginosa* and *S. aureus* it is surprising to note, that the highest efficiency, largest diameter, was observed for *P. aeruginosa,* a bacteria strain known to be resistant to common antibiotics, whereas *E.coli* showed the smallest increase in diameter. None of the bacteria strained tested was killed by the Ac-LK₆-NH₂ hydrogel controls, and thus the used Tris buffer seemed to have no influence at the bacteria.

To assess the bacteria growth inhibition in suspension OD measurements were carried out. For this purpose 24 well plates were coated with 400 µL Ag-Ac-LK₆-NH₂ hydrogels and cured overnight. At the same time Ac-LK₆-NH₂ hydrogels without silver were prepared as control. After the hydrogels were subjected to UV light to form the Ag NP 400 µL bacteria solution with an initial OD value of around 0.07 was added on top of the hydrogels. Tetracycline antibiotic, at a final concentration of 10 and 20 µg/mL was used as the positive control. Ac-LK₆-NH₂ hydrogels without Ag NPs and bacteria-only solutions served as the negative controls. After 24 h incubation the final OD value was determined and the results of the measurements can be found in Figure 6. In all three bacteria samples an OD smaller than the starting value was found for the samples treated with Ag-Ac-LK₆-NH₂ hydrogels, showing, that Ag-Ac-LK₆-NH₂ hydrogels are not only able to inhibit bacteria growth but also able to kill bacteria. In comparison, Ac-LK₆-NH₂ hydrogels alone only showed a small growth inhibition when compared to the untreated bacteria samples. Interestingly, the tetracycline controls showed similar activity as Ag-Ac-LK₆-NH₂ hydrogels in *E. coli* but were not as effective in *S. aureus.* In *P. aeruginosa* the largest difference between Ag-Ac-LK₆-NH₂ hydrogels and the tetracycline control was observed, where tetracycline only showed about 50% growth inhibition compared to the untreated control, whereas Ag-Ac-LK₆-NH₂ hydrogel was able to reduce the starting OD value.

### 2.5 Biocompatibility

Biocompatibility studies were carried out in 24-well transwell plates using 100 µL of gel in the transwell and 400 µL of medium in the peripheral well. Cell viability was measured after 24 h and 48 h of incubation. Ac-LK₆-NH₂ hydrogel without silver, 1 % agar, empty transwell and untreated cells were used as control and survival is presented as percentage normalized to the untreated cells (Figure 7A). After 24 h incubation a reduction in viability of about 20 % was found for all samples containing a transwell with hydrogels, independent of gel type in comparison to untreated cells. A similar result was found after 48 h incubation where all samples containing a hydrogel in the transwell showed a 20 % reduced viability compared to the untreated cells. However, it has to be pointed out, that Ag-Ac-LK₆-NH₂ hydrogel samples showed similar reduced viability to all other hydrogels containing control samples. In order to verify, that reduced cell viability is due to cell death live dead images were recorded after 48 h of incubation (Figure 7B-D). The superimposing of the fluorescence images did not show an increased amount of dead cells of the hydrogel samples when compared to the untreated control, which indicates, that the reduced viability is not due to the toxicity of the gels but rather due to a change in environment (transwell vs. no transwell).

### Applications

The disclosed hydrogels comprise metal nanoparticles and at least one peptide.

The disclosed hydrogels may be formed by the self-assembly of peptide(s) in water or physiologically relevant buffers.

The disclosed hydrogels may display superior mechanical strength. The metal nanoparticles may stabilize the fibrous structure of the hydrogel, therefore requiring a reduced concentration of peptide(s), which are considerably more expensive. Therefore, the disclosed hydrogels may be cost-effective.

The disclosed production process for the disclosed hydrogels is simple and does not require substantial chemical modifications.

The disclosed hydrogels may possess sustained anti-microbial activity over more than 10 days, are able to provide moist environment for cell regeneration over sustained period and are not adhesive.

The disclosed hydrogels may be user-friendly and may be applied over awkward or non-flat position with ease and with reduced frequency to change wound dressings.

### References

Adhikari B, Banerjee A. Short-Peptide-Based Hydrogel: A Template for the In Situ Synthesis of Fluorescent Silver Nanoclusters by Using Sunlight. Chemistry - A European Journal. 2010;16:13698-705.
Alt V, Bechert T, Steinrücke P, Wagener M, Seidel P, Dingeldein E, et al. An in vitro assessment of the antibacterial properties and cytotoxicity of nanoparticulate silver bone cement. Biomaterials. 2004;25:4383-91.
Brandt O, Mildner M, Egger AE, Groessl M, Rix U, Posch M, et al. Nanoscalic silver possesses broad-spectrum antimicrobial activities and exhibits fewer toxicological side effects than silver sulfadiazine. Nanomedicine : nanotechnology, biology, and medicine. 2012;8:478-88.
Fullenkamp DE, Rivera JG, Gong Y-k, Lau KHA, He L, Varshney R, et al. Mussel-inspired silver-releasing antibacterial hydrogels. Biomaterials. 2012;33:3783-91.
Hauser CAE, Deng R, Mishra A, Loo Y, Khoe U, Zhuang F, et al. Natural tri-to hexapeptides self-assemble in water to amyloid β-type fiber aggregates by unexpected α-helical intermediate structures. Proceedings of the National Academy of Sciences. 2011;108:1361-6.
Kim JS, Kuk E, Yu KN, Kim J-H, Park SJ, Lee HJ, et al. Antimicrobial effects of silver nanoparticles. Nanomedicine : nanotechnology, biology, and medicine. 2007;3:95-101.
Klasen HJ. Historical review of the use of silver in the treatment of burns. I. Early uses. Burns. 2000;26:117-30.
Klasen HJ. A historical review of the use of silver in the treatment of burns. II. Renewed interest for silver. Burns. 2000;26:131-8.
Lakshmanan A, Cheong DW, Accardo A, Di Fabrizio E, Riekel C, Hauser CAE. Aliphatic peptides show similar self-assembly to amyloid core sequences, challenging the importance of aromatic interactions in amyloidosis. Proceedings of the National Academy of Sciences. 2013;110:519-24.
Leaper DJ. Silver dressings: their role in wound management. International Wound Journal. 2006;3:282-94.
Martinez-Gutierrez F, Olive PL, Banuelos A, Orrantia E, Nino N, Sanchez EM, et al. Synthesis, characterization, and evaluation of antimicrobial and cytotoxic effect of silver and titanium nanoparticles. Nanomedicine : nanotechnology, biology, and medicine. 2010;6:681-8.
Martinez-Gutierrez F, Thi EP, Silverman JM, de Oliveira CC, Svensson SL, Hoek AV, et al. Antibacterial activity, inflammatory response, coagulation and cytotoxicity effects of silver nanoparticles. Nanomedicine : nanotechnology, biology, and medicine. 2012;8:328-36.
Mishra A, Loo Y, Deng R, Chuah YJ, Hee HT, Ying JY, et al. Ultrasmall natural peptides self-assemble to strong temperature-resistant helical fibers in scaffolds suitable for tissue engineering. Nano Today. 2011;6:232-9.
Murali Mohan Y, Vimala K, Thomas V, Varaprasad K, Sreedhar B, Bajpai SK, et al. Controlling of silver nanoparticles structure by hydrogel networks. Journal of Colloid and Interface Science. 2010;342:73-82.
Roy S, Banerjee A. Amino acid based smart hydrogel: formation, characterization and fluorescence properties of silver nanoclusters within the hydrogel matrix. Soft Matter. 2011;7:5300-8.
Rujitanaroj P-o, Pimpha N, Supaphol P. Wound-dressing materials with antibacterial activity from electrospun gelatin fiber mats containing silver nanoparticles. Polymer. 2008;49:4723-32.
Varaprasad K, Mohan YM, Ravindra S, Reddy NN, Vimala K, Monika K, et al. Hydrogel-silver nanoparticle composites: A new generation of antimicrobials. Journal of Applied Polymer Science. 2010;115:1199-207.
Vimala K, Mohan YM, Sivudu KS, Varaprasad K, Ravindra S, Reddy NN, et al. Fabrication of porous chitosan films impregnated with silver nanoparticles: A facile approach for superior antibacterial application. Colloids and Surfaces B: Biointerfaces. 2010;76:248-58.
Wright JB, Lam K, Buret AG, Olson ME, Burrell RE. Early healing events in a porcine model of contaminated wounds: effects of nanocrystalline silver on matrix metalloproteinases, cell apoptosis, and healing. Wound Repair and Regeneration. 2002;10:141-51.

## Claims

1. A nanoparticle-containing hydrogel comprising
metal nanoparticles selected from the group consisting of Ag nanoparticles, Cu nanoparticles, CuO nanoparticles, Fe₂O₃ nanoparticles, Pt nanoparticles, Pd nanoparticles and combinations thereof; and
at least one peptide having the general formula:
***Z*-(X)ₐ-(Y)_{b}-*Z*'_{c}**
wherein
***Z*** is an N-terminal protecting group;
**X** is, at each occurrence, independently selected from the group consisting of aliphatic amino acids;
**Y** is, at each occurrence, independently selected from the group consisting of polar amino acids, wherein said polar amino acids have a polar group which is independently selected from a hydroxyl, an ether, an imido, an amido, an ester, an amino, a guanidino, a seleno, and a telluro group;
***Z'*** is a C-terminal protecting group;
**a** is an integer selected from 1 to 6, preferably 1 to 5;
**b** is 1 or 2, preferably 1; and
**c** is 0 or 1.

2. The nanoparticle-containing hydrogel according to claim 1, wherein the metal nanoparticles have a uniform size distribution in the hydrogel, preferably have a particle size of less than 1 µm, more preferably 10 to 20 nm.

3. The nanoparticle-containing hydrogel according to claim 1 or 2, wherein said aliphatic amino acids, and said polar amino acids are independently selected from the group consisting of:
• either D-amino acids or L-amino acids;
• alanine (Ala, A), homoallylglycine, homopropargylglycine, isoleucine (Ile, I), norleucine, leucine (Leu, L), valine (Val, V) and glycine (Gly, G), preferably from the group consisting of alanine (Ala, A), isoleucine (Ile, I), leucine (Leu, L), valine (Val, V) and glycine (Gly, G), wherein all or a portion of said aliphatic amino acids are arranged in an order of decreasing amino acid size in the direction from N- to C-terminus, wherein the size of the aliphatic amino acids is defined as I = L > V > A > G; and
• LIVAG (SEQ ID NO: 1), ILVAG (SEQ ID NO: 2), LIVAA (SEQ ID NO: 3), IVAG (SEQ ID NO: 5), LIVA (SEQ ID NO: 6), LIVG (SEQ ID NO: 7), IVA (SEQ ID NO: 23) and IV (SEQ ID NO: 24), wherein, optionally, there is an A preceding such sequence at the N-terminus.

4. The nanoparticle-containing hydrogel according to any one of claims 1 to 3, wherein said polar amino acids are selected from the group consisting of asparagine (Asn, N), glutamine (Gln, Q), 5-N-ethyl-glutamine (theanine), citrulline, serine (Ser, S), homoserine, ornithine (Orn), threonine (Thr, T), methionine, L-Dopa, tryptophan (Trp, W), thyroxine, allo-threonine, lysine (Lys, K), 2,4-diaminobutyric acid (Dab), 2,3-diaminopropionic acid (Dap), and tyrosine (Tyr, Y), wherein said polar amino acid is preferably selected from the group consisting of asparagine, glutamine, serine, threonine, methionine, lysine, ornithine (Orn), 2,4-diaminobutyric acid (Dab), and 2,3-diaminopropionic acid (Dap).

5. The nanoparticle-containing hydrogel according to any one of claims 1 to 4, wherein (X)ₐ-(Y)_{b} has a sequence selected from the group consisting of LIVAGK (SEQ ID NO: 14), ILVAGK (SEQ ID NO. 15), LIVAGT (SEQ ID NO: 16), AIVAGT (SEQ ID NO: 17), IIIK (SEQ ID NO: 22), and IY (SEQ ID NO: 39).

6. The nanoparticle-containing hydrogel according to any one of claims 1 to 5, wherein said N-terminal protecting group ***Z*** is selected from group consisting of:
• general formula -C(O)-R, wherein R is selected from the group consisting of H, unsubstituted or substituted alkyls, and unsubstituted or substituted aryls or R is selected from the group consisting of methyl, ethyl, propyl, isopropyl, butyl and isobutyl;
• an acetyl group; and
• a peptidomimetic molecule, including natural and synthetic amino acid derivatives, wherein the N-terminus of said peptidomimetic molecule may be modified with a functional group selected from the group consisting of amide, alcohol, aldehyde, amine, imine, nitrile, an urea analog, phosphate, carbonate, sulfate, nitrate, maleimide, vinyl sulfone, azide, alkyne, alkene, carbohydrate, imide, peroxide, ester, aryl, ketone, sulphite, nitrite, phosphonate, and silane.

7. The nanoparticle-containing hydrogel according to any one of claims 1 to 6, wherein said C-terminal protecting group ***Z'*** is selected from the group consisting of:
• an amide group, wherein, preferably, the C-terminus has the formula - CONHR or -CONRR', with R and R' being selected from the group consisting of H, unsubstituted or substituted alkyls, and unsubstituted or substituted aryls;
• an ester group, wherein, preferably, the C-terminus has the formula - CO₂R, with R being selected from the group consisting of unsubstituted or substituted alkyls, and unsubstituted or substituted aryls; and
• a peptidomimetic molecule, including natural and synthetic amino acid derivatives, wherein the C-terminus of said peptidomimetic molecule is optionally modified with a functional group selected from the group consisting amide, alcohol, aldehyde, amine, imine, nitrile, an urea analog, phosphate, carbonate, sulfate, nitrate, maleimide, vinyl sulfone, azide, alkyne, alkene, carbohydrate, imide, peroxide, ester, aryl, ketone, sulphite, nitrite, phosphonate, and silane.

8. The nanoparticle-containing hydrogel according to any one of claims 1 to 7, wherein a is an integer selected from 1 to 5, preferably 2 to 5, and wherein, preferably, said aliphatic amino acids exhibit an overall decrease in hydrophobicity from the N-terminus to the C-terminus of said peptide.

9. The nanoparticle-containing hydrogel according to any one of claims 1 to 8, wherein said peptide is present at a concentration in the range of from 0.1 % to 30 % (w/w), preferably 0.1 % to 20 % (w/w), more preferably 0.1 % to 10 % (w/w), more preferably 0.1 % to 5 % (w/w), even more preferably 0.1 % to 3 % (w/w), with respect to the total weight of said nanoparticle-containing hydrogel.

10. The nanoparticle-containing hydrogel according to any one of claims 1 to 9, further comprising at least one additional substance, wherein said at least one additional substance is encapsulated by said hydrogel, immobilized in the bulk phase of said hydrogel or conjugated to said peptide, and wherein said at least one additional substance is selected from the group consisting of dyes, pigments, quantum dot nanoparticles and semiconductor nanoparticles.

11. An *in situ* method of producing a nanoparticle-containing hydrogel according to any one of claims 1 to 10, said method comprising the steps of
(a) dissolving at least one peptide as defined in any one of claims 1 and 3 to 10 in an aqueous solution;
(b) adding a precursor of metal nanoparticles, preferably a metal salt solution, wherein the metal salt is selected from AgNO₃, CuSO₄, CuSO₄•5H₂O, Cu(NO₃)₂ and its hydrates, Cu(OAc)₂ and its hydrates, FeCl₃ and its hydrates, FeCl₂ and its hydrates, K₂PtCl₄, PdCl₂₎ H₂PdCl₄ and Pd(NO₃)₂;
(c) gel formation to obtain a peptide hydrogel;
(d) irradiating the peptide hydrogel resulting from step (c); and
(e) obtaining a nanoparticle-containing hydrogel.

12. The method of claim 11, wherein the concentration of said metal salt precursor is in the range from 0.001 to 1M, preferably 1 to 100mM, even more preferred 10 to 50mM.

13. The method of any one of claims 11 or 12, wherein step (b) is carried out in water or in chloride and phosphate free buffer, preferably Tris buffer, either at physiological or at a basic pH, such as pH of about 7.4 or 8.5 (or from about pH 7 to about pH 9).

14. The method of claim 11, wherein said method comprises the steps of
(a) dissolving at least one peptide as defined in any one of claims 1 and 3 to 10 in an aqueous solution;
(b) adding a precursor of metal nanoparticles, preferably a metal salt solution, wherein the metal salt is selected from AgNO₃, CuSO₄, CuSO₄•5H₂O, Cu(NO₃)₂ and its hydrates, Cu(OAc)₂ and its hydrates, FeCl₃ and its hydrates, FeCl₂ and its hydrates, K₂PtCl₄, PdCl₂, H₂PdCl₄ and Pd(NO₃)₂;
(c) gel formation to obtain a peptide hydrogel;
(d) forming metal nanoparticles *in situ* through irradiation with light, preferably UV light, for minutes to hours, preferably 1 to 60 minutes, even more preferred 1 to 10 minutes, wherein the *in situ* reduction is achieved through a reducing agent, preferably a biocompatible reducing agent, more preferably, citric acid or sodium ascorbate, wherein the reducing agent is added before or after gelation (i.e. before or during step (c) or after (c)).

15. Use of a nanoparticle-containing hydrogel of any one of claims 1 to 10
- for bioimaging (by utilizing the fluorescence of the metal nanoparticles);
- for application where the hydrogel is used as a (dried) membrane, such as filtration; or
- in a cosmetic composition.

16. A bioimaging device comprising a nanoparticle-containing hydrogel of any one of claims 1 to 10 for *in vitro* and/or *in vivo* use, preferably for oral application, for injection and/or for topical application.

17. A nanoparticle-containing hydrogel of any one of claims 1 to 10 for use in treating an infectious disease, in preventing bacterial attachment and/or formation of microbial biofilm, in combination with medical devices, such as stents, prostheses, catheters, dental implants, orthopedical devices, or orthopedical implants, or in a pharmaceutical composition.

## Patentansprüche

1. Nanopartikelhaltiges Hydrogel, umfassend:
Metallnanopartikel, ausgewählt aus der Gruppe, bestehend aus Ag-Nanopartikeln, Cu-Nanopartikeln, CuO-Nanopartikeln, Fe₂O₃-Nanopartikeln, Pt-Nanopartikeln, Pd-Nanopartikeln und
Kombinationen davon; und
mindestens ein Peptid mit der folgenden allgemeinen Formel:
***Z*-(X)ₐ-(Y)_{b}-*Z*'_{c}**
wobei
***Z*** eine N-terminale Schutzgruppe ist;
**X** bei jedem Auftreten unabhängig aus der Gruppe ausgewählt ist,
bestehend aus aliphatischen Aminosäuren;
**Y** bei jedem Auftreten unabhängig aus der Gruppe ausgewählt ist, bestehend aus polaren Aminosäuren, wobei die polaren Aminosäuren eine polare Gruppe aufweisen, die unabhängig aus einer Hydroxyl-, einer Ether-, einer Imido-, einer Amido-, einer Ester-, einer Amino-, einer Guanidino-, einer Seleno- und einer Tellurogruppe ausgewählt ist;
***Z'*** eine C-terminale Schutzgruppe ist;
**a** eine ganze Zahl ist, ausgewählt aus 1 bis 6, vorzugsweise 1 bis 5;
**b** 1 oder 2, vorzugsweise 1 ist; und
**c** 0 oder 1 ist.

2. Nanopartikelhaltiges Hydrogel nach Anspruch 1, wobei die Metallnanopartikel in dem Hydrogel eine gleichmäßige Größenverteilung aufweisen, vorzugsweise eine Partikelgröße kleiner gleich 1 µm, vorzugsweiser 10 bis 20 nm, aufweisen.

3. Nanopartikelhaltiges Hydrogel nach Anspruch 1 oder 2, wobei die aliphatischen Aminosäuren und die polaren Aminosäuren unabhängig aus der Gruppe ausgewählt werden, bestehend aus:
• entweder D-Aminosäuren oder L-Aminosäuren;
• Alanin (Ala, A), Homoallylglycin, Homopropargylglycin, Isoleucin (Ile, I), Norleucin, Leucin (Leu, L), Valin (Val, V) und Glycin (Gly, G), vorzugsweise aus der Gruppe, bestehend aus Alanin (Ala, A), Isoleucin (Ile, I), Leucin (Leu, L), Valin (Val, V) und Glycin (Gly, G), wobei alle oder ein Teil der aliphatischen Aminosäuren in einer Reihenfolge abnehmender Aminosäuregröße in der Richtung vom N- zum C-Terminus angeordnet sind, wobei die Größe der aliphatischen Aminosäuren als I = L > V> A > G definiert ist; und
• LIVAG (SEQ ID NO: 1), ILVAG (SEQ ID NO: 2), LIVAA (SEQ ID NO: 3), IVAG (SEQ ID NO: 5), LIVA (SEQ ID NO: 6), LIVG (SEQ ID NO: 7), IVA (SEQ ID NO: 23) und IV (SEQ ID NO: 24), wobei es gegebenenfalls ein A gibt, das einer solchen Sequenz an dem N-Terminus vorausgeht.

4. Nanopartikelhaltiges Hydrogel nach einem der Ansprüche 1 bis 3, wobei die polaren Aminosäuren aus der Gruppe ausgewählt werden, bestehend aus Asparagin (Asn, N), Glutamin (Gln, Q), 5-N-Ethyl-Glutamin (Theanin), Citrullin, Serin (Ser, S), Homoserin, Ornithin (Orn), Threonin (Thr, T), Methionin, L-Dopa, Tryptophan (Trp, W), Thyroxin, allo-Threonin, Lysin (Lys, K), 2,4-Diaminobuttersäure (Dab), 2,3-Diaminopropionsäure (Dap) und Tyrosin (Tyr, Y),
wobei die polare Aminosäure vorzugsweise aus der Gruppe ausgewählt ist, bestehend aus Asparagin, Glutamin, Serin, Threonin, Methionin, Lysin, Ornithin (Orn), 2,4-Diaminobuttersäure (Dab) und 2,3-Diaminopropionsäure (Dap).

5. Nanopartikelhaltiges Hydrogel nach einem der Ansprüche 1 bis 4, wobei **(X)ₐ-(Y)_{b}** eine Sequenz aufweist, ausgewählt aus der Gruppe, bestehend aus LIVAGK (SEQ ID NO: 14), ILVAGK (SEQ ID NO. 15), LIVAGT (SEQ ID NO: 16), AIVAGT (SEQ ID NO: 17), **IIIK (SEQ ID NO: 22)** und IY (SEQ ID NO: 39).

6. Nanopartikelhaltiges Hydrogel nach einem der Ansprüche 1 bis 5, wobei die N-terminale Schutzgruppe ***Z*** aus der Gruppe ausgewählt ist, bestehend aus:
• der allgemeinen Formel **-C(O)-R,** wobei R aus der Gruppe ausgewählt ist, bestehend aus H, unsubstituierten oder substituierten Alkylen, und unsubstituierten oder substituierten Arylen oder R aus der Gruppe ausgewählt ist, bestehend aus Methyl, Ethyl, Propyl, Isopropyl, Butyl und Isobutyl;
• einer Acetylgruppe; und
• einem peptidomimetischen Molekül, einschließend natürliche und synthetische Aminosäurederivate, wobei der N-Terminus des peptidomimetischen Moleküls mit einer funktionellen Gruppe modifiziert werden kann, ausgewählt aus der Gruppe, bestehend aus Amid, Alkohol, Aldehyd, Amin, Imin, Nitril, einem Harnstoffanalogon, Phosphat, Carbonat, Sulfat, Nitrat, Maleimid, Vinylsulfon, Azid, Alkin, Alken, Kohlenhydrat, Imid, Peroxid, Ester, Aryl, Keton, Sulfit, Nitrit, Phosphonat und Silan.

7. Nanopartikelhaltiges Hydrogel nach einem der Ansprüche 1 bis 6, wobei die C-terminale Schutzgruppe ***Z'*** aus der Gruppe ausgewählt ist, bestehend aus:
• einer Amidgruppe, wobei der C-Terminus vorzugsweise die Formel -CONHR oder **-CONRR'** aufweist, wobei R und R' aus der Gruppe ausgewählt sind, bestehend aus H, unsubstituierten oder substituierten Alkylen, und unsubstituierten oder substituierten Arylen;
• einer Estergruppe, wobei der C-Terminus vorzugsweise die Formel -CO₂R aufweist, wobei R aus der Gruppe ausgewählt ist, bestehend aus unsubstituierten oder substituierten Alkylen, und unsubstituierten oder substituierten Arylen; und
• einem peptidomimetischen Molekül, einschließend natürliche und synthetische Aminosäurederivate, wobei der C-Terminus des peptidomimetischen Moleküls gegebenenfalls mit einer funktionellen Gruppe modifiziert ist, ausgewählt aus der Gruppe, bestehend aus Amid, Alkohol, Aldehyd, Amin, Imin, Nitril, einem Harnstoffanalogon, Phosphat, Carbonat, Sulfat, Nitrat, Maleimid, Vinylsulfon, Azid, Alkin, Alken, Kohlenhydrat, Imid, Peroxid, Ester, Aryl, Keton, Sulfit, Nitrit, Phosphonat und Silan.

8. Nanopartikelhaltiges Hydrogel nach einem der Ansprüche 1 bis 7, wobei **a** eine ganze Zahl ist, ausgewählt aus 1 bis 5, vorzugsweise 2 bis 5, und wobei die aliphatischen Aminosäuren von dem N-Terminus zum C-Terminus des Peptids vorzugsweise einen Gesamtrückgang der Hydrophobizität aufweisen.

9. Nanopartikelhaltiges Hydrogel nach einem der Ansprüche 1 bis 8, wobei das Peptid in Bezug auf das Gesamtgewicht des nanopartikelhaltigen Hydrogels mit einer Konzentration in dem Bereich von 0,1 Gew.-% bis 30 Gew.-%, vorzugsweise 0,1 Gew.-% bis 20 Gew.-%, vorzugsweiser 0,1 Gew.-% bis 10 Gew.-%, vorzugsweiser 0,1 Gew.-% bis 5 Gew.-%, sogar noch vorzugsweiser 0,1 Gew.-% bis 3 Gew.-%, vorliegt.

10. Nanopartikelhaltiges Hydrogel nach einem der Ansprüche 1 bis 9, ferner umfassend mindestens eine zusätzliche Substanz, wobei die mindestens eine zusätzliche Substanz von dem Hydrogel verkapselt, in der umgebenden Phase des Hydrogels immobilisiert oder an das Peptid konjugiert wird, und wobei die mindestens eine zusätzliche Substanz aus der Gruppe ausgewählt ist, bestehend aus Farbstoffen, Pigmenten, Quantenpunktnanopartikeln und Halbleiternanopartikeln.

11. *In-situ*-Verfahren zum Herstellen eines nanopartikelhaltigen Hydrogels nach einem der Ansprüche 1 bis 10, wobei das Verfahren die folgenden Schritte umfasst:
(a) Auflösen von mindestens einem Peptid nach einem der Ansprüche 1 und 3 bis 10 in einer wässrigen Lösung;
(b) Hinzugeben eines Vorläufers von Metallnanopartikeln, vorzugsweise eine Metallsalzlösung, wobei das Metallsalz aus **AgNO₃, CuSO₄, CuSO₄•5H₂O, Cu(NO₃)₂** und seinen Hydraten, **Cu(OAc)₂** und seinen Hydraten, FeCl₃ und seinen Hydraten, FeCl₂ und seinen Hydraten, **K₂PtCl₄, PdCl₂, H₂PdCl₄** und **Pd(NO₃)₂** ausgewählt ist;
(c) Gelbildung, um ein Peptidhydrogel zu erhalten;
(d) Bestrahlen des aus Schritt (c) hervorgegangenen Hydrogels; und
(e) Erhalten eines nanopartikelhaltigen Hydrogels.

12. Verfahren nach Anspruch 11, wobei die Konzentration des Metallsalzvorläufers in dem Bereich von 0,001 bis 1 M, vorzugsweise 1 bis 100 mM, sogar noch bevorzugter 10 bis 50 mM liegt.

13. Verfahren nach einem der Ansprüche 11 oder 12, wobei Schritt (b) in Wasser oder in chlorid- und phosphatfreiem Puffer, vorzugsweise Tris-Puffer, entweder bei einem physiologischen oder einem basischen pH-Wert, wie etwa einem pH-Wert von ungefähr 7,4 oder 8,5 (oder von ungefähr pH 7 bis ungefähr pH 9) ausgeführt wird.

14. Verfahren nach Anspruch 11, wobei das Verfahren die folgenden Schritte umfasst:
(a) Auflösen von mindestens einem Peptid nach einem der Ansprüche 1 und 3 bis 10 in einer wässrigen Lösung;
(b) Hinzugeben eines Vorläufers von Metallnanopartikeln, vorzugsweise eine Metallsalzlösung, wobei das Metallsalz aus **AgNO₃, CuSO₄, CuSO₄•5H₂O, Cu(NO₃)₂** und seinen Hydraten, **Cu(OAc)₂** und seinen Hydraten, FeCl₃ und seinen Hydraten, FeCl₂ und seinen Hydraten, **K₂PtCl₄, PdCl₂, H₂PdCl₄** und **Pd(NO₃)₂** ausgewählt ist;
(c) Gelbildung, um ein Peptidhydrogel zu erhalten;
(d) Bilden von Metallnanopartikeln *in situ* durch eine Bestrahlung mit Licht, vorzugsweise UV-Licht, für Minuten bis Stunden, vorzugsweise 1 bis 60 Minuten, sogar noch bevorzugter 1 bis 10 Minuten, wobei die Reduktion *in situ* durch ein Reduktionsmittel, vorzugsweise ein biokompatibles Reduktionsmittel, vorzugsweiser Citronensäure oder Natriumascorbat, erreicht wird,
wobei das Reduktionsmittel vor oder nach dem Gelieren (d. h. vor oder während des Schritt (c) oder nach (c)) hinzugegeben wird.

15. Verwendung eines nanopartikelhaltigen Hydrogels nach einem der Ansprüche 1 bis 10
- für die biologische Bildgebung (unter Verwendung der Fluoreszenz der Metallnanopartikel);
- zur Anwendung, wobei das Hydrogel als eine (getrocknete) Membran verwendet wird, wie etwa Filtration;
oder
- in einer kosmetischen Zusammensetzung.

16. Vorrichtung zur biologischen Bildgebung, umfassend ein nanopartikelhaltiges Hydrogel nach einem der Ansprüche 1 bis 10 zur Verwendung *in vitro* und/oder *in vivo,* vorzugsweise zur oralen Anwendung, zur Injektion und/oder zur topischen Anwendung.

17. Nanopartikelhaltiges Hydrogel nach einem der Ansprüche 1 bis 10 zur Verwendung bei der Behandlung einer Infektionskrankheit, beim Verhindern der Bakterienanheftung und/oder bei der Bildung eines mikrobiellen Biofilms in Kombination mit medizinischen Vorrichtungen, wie etwa Stents, Prothesen, Kathetern, Zahnimplantaten, orthopädischen Vorrichtungen, oder orthopädischen Implantaten, oder in einer pharmazeutischen Zusammensetzung.

## Revendications

1. Hydrogel contenant des nanoparticules comprenant des nanoparticules de métal choisies dans le groupe constitué des nanoparticules d'Ag, des nanoparticules de Cu, des nanoparticules de CuO, des nanoparticules de Fe₂O₃, des nanoparticules de Pt, des nanoparticules de Pd et de combinaisons de celles-ci ; et
au moins un peptide de formule générale :
***Z-*(X)ₐ-(Y)_{b}-*Z*'_{c}**
où
*Z* est un groupe protecteur en terminaison N ;
**X** est, à chaque occurrence, indépendamment choisi dans le groupe constitué d'acides aminés aliphatiques ;
**Y** est, à chaque occurrence, indépendamment choisi dans le groupe constitué d'acides aminés polaires, dans lequel lesdits acides aminés polaires ont un groupe polaire qui est indépendamment choisi parmi un hydroxyle, un éther, un imido, un amido, un ester, un amino, un guanidino, un seleno et un groupe tellure ;
***Z'*** est un groupe protecteur en terminaison C ;
**a** est un entier choisi entre 1 et 6, de préférence entre 1 et 5 ;
**b** est égal à 1 ou à 2, de préférence 1 ; et
**c** est égal à 0 ou à 1.

2. Hydrogel contenant des nanoparticules selon la revendication 1, dans lequel les nanoparticules de métal ont une distribution de taille uniforme dans l'hydrogel, ont de préférence une taille de particules inférieure à 1 µm, de manière davantage préférée de 10 à 20 nm.

3. Hydrogel contenant des nanoparticules selon les revendications 1 ou 2, dans lequel lesdits acides aminés aliphatiques et lesdits acides aminés polaires sont choisis indépendamment dans le groupe constitué :
• soit des acides aminés D, soit des acides aminés L ;
• de l'alanine (Ala, A), de l'homoallylglycine, de l'homopropargylglycine, de l'isoleucine (Ile, I), de la norleucine, de la leucine (Leu, L), de la valine (Val, V) et de la glycine (Gly, G), de préférence dans le groupe constitué de l'alanine (Ala, A), de l'isoleucine (Ile, I), de la leucine (Leu, L), de la valine (Val, V) et de la glycine (Gly, G), dans lequel la totalité ou une partie desdits acides aminés aliphatiques sont disposés dans un ordre de taille d'acide aminé décroissante de la terminaison N en direction de la terminaison C, dans lequel la taille des acides aminés aliphatiques est définie comme I = L > V > A > G ; et
• de LIVAG (SEQ ID NO: 1), d'ILVAG (SEQ ID NO: 2), de LIVAA (SEQ ID NO: 3), d'IVAG (SEQ ID NO: 5), de LIVA (SEQ ID NO: 6), de LIVG (SEQ ID NO : 7), d'IVA (SEQ ID NO: 23) et d'IV (SEQ ID NO: 24), dans lequel, éventuellement, un A précède une telle séquence à la terminaison N.

4. Hydrogel contenant des nanoparticules selon l'une quelconque des revendications 1 à 3, dans lequel lesdits acides aminés polaires sont choisis dans le groupe constitué de l'asparagine (Asn, N), de la glutamine (Gin, Q), de la 5-N-éthyl-glutamine (théanine), de la citrulline, de la sérine (Ser, S), de l'homosérine, de l'ornithine (Orn), de la thréonine (Thr, T), de la méthionine, du L-Dopa, du tryptophane (Trp, W), de la thyroxine, de l'allo-thréonine, de la lysine (Lys, K), de l'acide 2,4-diaminobutyrique (Dab), de l'acide 2,3-diaminopropionique (Dap), et de la tyrosine (Tyr, Y), dans lequel ledit acide aminé polaire est de préférence choisi dans le groupe constitué de l'asparagine, de la glutamine, de la sérine, de la thréonine, de la méthionine, de la lysine, de l'ornithine (Orm), de l'acide 2,4-diaminobutyrique (Dab) et de l'acide 2,3-diaminopropionique (Dap).

5. Hydrogel contenant des nanoparticules selon l'une quelconque des revendications 1 à 4, dans lequel (X)ₐ-(Y)_{b} a une séquence sélectionnée dans le groupe constitué de LIVAGK (SEQ ID NO: 14), d'ILVAGK (SEQ ID NO. 15), de LIVAGT (SEQ ID NO: 16), d'AIVAGT (SEQ ID NO: 17), d'IIIK (SEQ ID NO: 22) et d'IY (SEQ ID NO: 39).

6. Hydrogel contenant des nanoparticules selon l'une quelconque des revendications 1 à 5, dans lequel ledit groupe protecteur en terminaison N ***Z*** est choisi dans le groupe constitué :
• de la formule générale -C(O)-R, dans lequel R est choisi dans le groupe constitué de H, d'alkyles non substitués ou substitués et d'aryles non substitués ou substitués ou R est choisi dans le groupe constitué du méthyle, de l'éthyle, du propyle, de l'isopropyle, du butyle et de l'isobutyle ;
• d'un groupe acétyle ; et
• d'une molécule peptidomimétique, comprenant des dérivés d'acides aminés naturels et synthétiques, dans lequel la terminaison N de ladite molécule peptidomimétique peut être modifiée avec un groupe fonctionnel choisi dans le groupe constitué de l'amide, de l'alcool, de l'aldéhyde, de l'amine, de l'imine, du nitrile, d'un analogue de l'urée, du phosphate, du carbonate, du sulfate, du nitrate, du maléimide, du vinylsulfone, de l'azide, de l'alcyne, de l'alcène, du glucide, de l'imide, du peroxyde, de l'ester, de l'aryle, du cétone, du sulfite, du nitrite, du phosphonate et du silane.

7. Hydrogel contenant des nanoparticules selon l'une quelconque des revendications 1 à 6, dans lequel ledit groupe protecteur en terminaison C **Z'** est choisi dans le groupe constitué :
• d'un groupe amide, dans lequel, de préférence, la terminaison C a la formule -CONHR ou -CONRR' , R et R' étant choisis dans le groupe constitué de H, des alkyles non substitués ou substitués et des aryles non substitués ou substitués ;
• d'un groupe ester, dans lequel, de préférence, la terminaison C a la formule -CO₂R, R étant choisi dans le groupe constitué des alkyles non substitués ou substitués, et des aryles non substitués ou substitués ; et
• d'une molécule peptidomimétique, comprenant des dérivés d'acides aminés naturels et synthétiques, dans lequel la terminaison C de ladite molécule peptidomimétique est éventuellement modifiée avec un groupe fonctionnel choisi dans le groupe constitué de l'amide, de l'alcool, de l'aldéhyde, de l'amine, de l'imine, du nitrile, d'un analogue de l'urée, du phosphate, du carbonate, du sulfate, du nitrate, du maléimide, du vinylsulfone, de l'azide, de l'alcyne, de l'alcène, du glucide, de l'imide, du peroxyde, de l'ester, de l'aryle, du cétone, du sulfite, du nitrite, du phosphonate et du silane.

8. Hydrogel contenant des nanoparticules selon l'une quelconque des revendications 1 à 7, dans lequel a est un nombre entier choisi entre 1 et 5, de préférence entre 2 et 5, et dans lequel, de préférence, lesdits acides aminés aliphatiques présentent une diminution générale de l'hydrophobicité de la terminaison N à la terminaison C dudit peptide.

9. Hydrogel contenant des nanoparticules selon l'une quelconque des revendications 1 à 8, dans lequel ledit peptide est présent à une concentration comprise dans la plage de 0,1 % à 30 % (p/p), de préférence de 0,1 % à 20 % (p/p), de manière davantage préférée de 0,1 % à 10 % (p/p), de manière davantage préférée de 0, 1 % à 5 % (p/p), et de manière davantage préférée encore de 0,1 % à 3 % (p/p), par rapport au poids total dudit hydrogel contenant des nanoparticules.

10. Hydrogel contenant des nanoparticules selon l'une quelconque des revendications 1 à 9, comprenant en outre au moins une substance supplémentaire, dans lequel ladite au moins une substance supplémentaire est encapsulée par ledit hydrogel, immobilisée dans la phase en vrac dudit hydrogel ou conjuguée audit peptide, et dans lequel ladite au moins une substance supplémentaire est choisie dans le groupe constitué de colorants, de pigments, de nanoparticules à points quantiques et de nanoparticules semi-conductrices.

11. Procédé *in situ* de production d'un hydrogel contenant des nanoparticules selon l'une quelconque des revendications 1 à 10, ledit procédé comprenant les étapes
(a) de dissolution d'au moins un peptide selon l'une quelconque des revendications 1 et 3 à 10 dans une solution aqueuse ;
(b) d'ajout d'un précurseur de nanoparticules de métal, de préférence d'une solution de sel métallique, dans lequel le sel métallique est choisi parmi AgNO₃, CuSO₄, CuSO₄·5H₂O, Cu(NO₃)₂ et ses hydrates, Cu(OAc)₂ et ses hydrates, FeCl₃ et ses hydrates, FeCl₂ et ses hydrates, K₂PtCl₄, PdCl₂, H₂PdCl₄ et Pd(NO₃)₂;
(c) de formation de gel pour obtenir un hydrogel peptidique ;
(d) d'irradiation de l'hydrogel peptidique résultant de l'étape (c) ; et
(e) d'obtention d'un hydrogel contenant des nanoparticules.

12. Procédé selon la revendication 11, dans lequel la concentration dudit précurseur de sel métallique est comprise dans la plage de 0,001 à 1M, de préférence de 1 à 100 mM, de manière davantage préférée encore de 10 à 50 mM.

13. Procédé selon l'une quelconque des revendications 11 ou 12, dans lequel l'étape (b) est effectuée dans l'eau ou dans un tampon sans chlorure ni phosphate, de préférence un tampon Tris, soit à un pH physiologique soit à un pH basique, tel qu'un pH d'environ 7,4 ou 8,5 (ou un pH d'environ 7 à environ pH 9).

14. Procédé selon la revendication 11, dans lequel ledit procédé comprend les étapes
(a) de dissolution d'au moins un peptide selon l'une quelconque des revendications 1 et 3 à 10 dans une solution aqueuse ;
(b) d'ajout d'un précurseur de nanoparticules de métal, de préférence d'une solution de sel métallique, dans lequel le sel métallique est choisi parmi AgNO₃, CuSO₄, CuS0₄^{∗}5H₂O, Cu(NO₃)₂ et ses hydrates, Cu(OAc)₂ et ses hydrates, FeCl₃ et ses hydrates, FeCl₂ et ses hydrates, K₂PtCl₄, PdCl₂, H₂PdCl₄ et Pd(NO₃)₂ ;
(c) de formation de gel pour obtenir un hydrogel peptidique ;
(d) de formation de nanoparticules de métal *in situ* par irradiation à la lumière, de préférence à la lumière UV, pendant plusieurs minutes à plusieurs heures, de préférence 1 à 60 minutes, de manière encore davantage préférée 1 à 10 minutes, dans lequel la réduction *in situ* est obtenue grâce à un agent réducteur, de préférence un agent réducteur biocompatible, de manière davantage préférée, de l'acide citrique ou de l'ascorbate de sodium, dans lequel l'agent réducteur est ajouté avant ou après la gélification (c'est-à-dire avant ou pendant l'étape (c) ou après (c)).

15. Utilisation d'un hydrogel contenant des nanoparticules selon l'une quelconque des revendications 1 à 10
- pour la bioimagerie (en utilisant la fluorescence des nanoparticules de métal) ;
- pour une application où l'hydrogel est utilisé comme membrane (séchée), telle que la filtration ; ou
- dans une composition cosmétique.

16. Dispositif de bioimagerie comprenant un hydrogel contenant des nanoparticules selon l'une quelconque des revendications 1 à 10 pour une utilisation *in vitro* et/ou *in vivo,* de préférence pour une application orale, pour une injection et/ou pour une application topique.

17. Hydrogel contenant des nanoparticules selon l'une quelconque des revendications 1 à 10, destiné à être utilisé dans le traitement d'une maladie infectieuse, pour prévenir la fixation bactérienne et/ou la formation de biofilm microbien, en combinaison avec des dispositifs médicaux, tels que des stents, des prothèses, des cathéters, des implants dentaires, des dispositifs orthopédiques ou des implants orthopédiques, ou dans une composition pharmaceutique.
